# EUROPEAN PATENT APPLICATION

(11) **EP 4 471 150 A1**
(43) Date of publication of application: **04.12.2024**
(21) Application number: 23746314.6
(22) Date of filing: 20.01.2023
(51) Int. Cl.: C12N 15/86, C12N 15/864, A61K 48/00, A61P 25/28

(54) **RECOMBINANT VIRUS EXPRESSING TPK AND USE THEREOF IN TREATMENT OF ALZHEIMER'S DISEASE**

(30) Priority: 29.01.2022 CN 202210112858
(71) Applicant: Shanghai Raising Pharmaceutical Co., Ltd., Pudong New District Shanghai 201321 (CN)
(72) Inventor: WANG, Jingjing, Shanghai 201321 (CN); SANG, Shaoming, Shanghai 201321 (CN); ZHANG, Huan, Shanghai 201321 (CN)
(74) Representative: Nederlandsch Octrooibureau
(86) International application number: PCT/CN2023/073317
(87) International publication number: WO 2023/143435

(57) **Abstract**

Provided is a recombinant adeno-associated virus (rAAV) or recombinant lentivirus, comprising an expression cassette in the genome, the expression cassette comprises a polynucleotide encoding thiamine pyrophosphokinase (TPK), which is operatively linked to a promoter. Also provided are also a pharmaceutical composition comprising the rAAV or recombinant lentivirus, and use of the rAAV, recombinant lentivirus and the pharmaceutical composition in the preparation of a medicament for treating or preventing Alzheimer's disease.

## Description

### Technical Field

The present disclosure relates to the field of gene therapy. In particular, the present disclosure relates to a recombinant adeno-associated virus (rAAV) or recombinant lentivirus, comprising a nucleotide encoding thiamine pyrophosphokinase (TPK), wherein the rAAV or recombinant lentivirus is useful for treating Alzheimer's disease.

### Background

Alzheimer's disease (AD) is a severe neurodegenerative disease, which mainly occurs in the elderly. Occurrence of the disease is accompanied by a large number of neuronal apoptosis and leads to cerebral parenchymal atrophy. The main clinical features of this disease comprise memory impairment, decreased language ability and spatial orientation ability, and mental abnormality or the like. At present, the pathogenesis of AD remains unclear, and there are various hypotheses about the mechanism of AD, such as β-amyloid protein (Aβ) cascade hypothesis, phosphorylated Tau protein hypothesis, neuroinflammatory response hypothesis, and oxidative stress hypothesis. In the past decades, extensive researches have been conducted based on these hypotheses, but no effective therapeutic scheme has been achieved.

The brain accounts for about 2% of body weight, but the energy consumed accounts for about 20% of total energy consumed by the human body, indicating that glucose metabolism is crucial for normal function of the brain. It has been found in researches that abnormal glucose metabolism occurs in the brain of an AD patient prior to the clinical symptoms, and the level of glucose metabolism in the patient's brain gradually decreases as the disease progresses. Therefore, the regulation of the glucose metabolism level of brain cells is expected to alleviate or terminate the progression of AD.

Generally, the regulation of gene expression in cells can be achieved by the methods such as calcium transformation, liposome transfection, electroporation, and infection with transgenic virus.

Adeno-associated virus (AAV) is a single stranded DNA virus which is widely present in human, and its natural hosts are human and other primates. Currently, the report that AAV can be pathogenic has not been found. Recombinant adeno-associated virus (rAAV) is obtained through engineering on the basis of AAV, and has been widely used as the vector for gene therapy due to the high safety, wide host range (both dividing and non-dividing cells can be infected), and low immunogenicity.

FDA approved two rAAV-based gene therapy products Luxturna and Zolgensma in 2017 and 2019, respectively. Luxturna was jointly developed by Spark Therapeutics and Florida Children's Hospital for the treatment of Burkholder's congenital black cataract. AAV2 is used as vector in Luxturna, carrying human RPE65 cDNA, which is administered via subretinal injection. Zolgensma was developed by Novartis for the treatment of spinal muscular atrophy in children. AAV9 is used as vector in Zolgensma, carrying SMN1 gene necessary for motor neuron survival, which is administered via intravenous injection.

Vitamin B1, also known as thiamine (TM), is converted to biologically active thiamine diphosphate (TDP) by thiamine pyrophosphokinase (TPK) in the body. TDP is a necessary coenzyme for transketonase, pyruvate dehydrogenase, α-ketoglutarate dehydrogenase in glucose metabolism. It has been shown in research that the activity of transketonase is decreased by more than 45%, the activity of α-ketoglutarate dehydrogenase is decreased by more than 75% in the brain tissue of an AD patient; the TDP level in the whole blood of an AD patient is significantly lower than the normal level, and the TDP level in the whole blood is highly associated with the decrease of glucose metabolism in the brain of an AD patient. It has been reported in research that the expression levels of TPK mRNA and protein in the brain tissue of an AD patient are significantly lower than those in the brain tissue of normal human with matched age and gender. More importantly, the TPK mRNA level is positively associated in the brain of a subject at normal cognitive state, but is negatively associated in an AD patient, with the Braak score of AD pathological grading. The results indicate that TPK expression may be a protective factor that delays the disease occurrence and development of AD. Conditional knockout of TPK gene in excitatory neurons of mouse brain significantly leads to abnormal glucose metabolism and cognitive impairment, brain atrophy caused by synaptic and neuronal loss, Aβ sedimentation and plaque formation, Tau abnormal phosphorylation and neurofibrillary tangles formation, activation of microglia and astrocytes and neuroinflammation, micro-angiopoiesis disorder, and peripheral glucose metabolism regulation disorder in mouse, which are various important pathophysiological features of all human AD diseases (see, e.g., Sang et al., Thiamine pyrophosphokinase deficiency induces Alzheimer's pathology, bioRxiv, 2020; and WO2021023069A1).

At present, it is desirable to develop a new therapy for Alzheimer's disease, such as an rAAV based gene therapy, to overcome the current issue of drug shortage, such as an rAAV based gene therapy.

### Summary of the Invention

In the first aspect, provided is a recombinant adeno-associated virus (rAAV) or recombinant lentivirus, comprising in the genome an expression cassette comprising a polynucleotide encoding thiamine pyrophosphokinase (TPK) operatively linked to a promoter.

In some embodiments, the TPK comprises an amino acid sequence of SEQ ID NO: 1. In some embodiments, the polynucleotide comprises a nucleotide sequence of SEQ ID NO: 2.

In some embodiments, the promoter is a neuron-specific promoter. In some embodiments, the promoter comprises a nucleotide sequence of SEQ ID NO: 3.

In some embodiments, the expression cassette further comprises a WPRE element. In some embodiments, the WPRE element comprises a nucleotide sequence of SEQ ID NO: 4.

In some embodiments, the promoter is eukaryotic strong promoter, for example, cytomegalovirus (CMV) promoter. In some embodiments, the promoter comprises a nucleotide sequence of SEQ ID NO: 7.

In some embodiments, the rAAV is an rAAV of serotype AAV_PHP eB.

In the second aspect, provided is a modified thiamine pyrophosphokinase (TPK) polypeptide comprising the substitution of amino acid at one or more positions selected from the group consisting of positions 13, 30, 31, 37, 85, 129, 158, 181, 11 and 35, as compared to an initial TPK polypeptide, wherein the amino acid at position 13 is substituted with P, the amino acid at position 30 is substituted with A, the amino acid at position 31 is substituted with R, the amino acid at position 37 is substituted with K, the amino acid at position 85 is substituted with K, the amino acid at position 129 is substituted with G, the amino acid at position 158 is substituted with K, the amino acid at position 181 is substituted with S, the amino acid at position 11 is substituted with W, the amino acid at position 35 is substituted with W, wherein the modified TPK polypeptide has an improved catalytic activity of converting thiamine (TM) to thiamine diphosphate (TDP) as compared to the initial TPK polypeptide, and wherein the positions are numbered by reference to SEQ ID NO: 1 or 8.

In some embodiments, the initial TPK polypeptide is wild type TPK polypeptide. In some embodiments, the initial TPK polypeptide is human TPK polypeptide, and wherein the modified TPK polypeptide comprises the substitution of amino acid at one or more positions selected from the group consisting of positions 13, 30, 31, 37, 85, 129, 158 and 181, preferably all of positions 13, 30, 31, 37, 85, 129, 158 and 181. In some embodiments, the initial TPK polypeptide is mouse TPK polypeptide, and the modified TPK polypeptide comprises the modified TPK polypeptide comprises the substitution of amino acid at positions 11 and/or 35. In some embodiments, the modified TPK polypeptide comprises an amino acid sequence of SEQ ID NO: 9, 10 or 11.

In the third aspect, provided is a polynucleotide, encoding the modified TPK polypeptide according to the present disclosure.

Provided is also an expression cassette, comprising the polynucleotide according to the present disclosure, operatively linked to a promoter. In some embodiments, the promoter is a neuron-specific promoter. In some embodiments, the promoter comprises a nucleotide sequence of SEQ ID NO: 3. In some embodiments, the expression cassette further comprises a WPRE element. In some embodiments, the WPRE element comprises a nucleotide sequence of SEQ ID NO: 4.

In the fourth aspect, provided is an rAAV or recombinant lentivirus, comprising in the genome the expression cassette as provided in the third aspect according to the present disclosure.

In some embodiments, the rAAV is a rAAV of serotype AAV_PHP eB.

In the fifth aspect, provided is a pharmaceutical composition, comprising the modified TPK polypeptide, polynucleotide, rAAV or recombinant lentivirus according to the present disclosure, and a pharmaceutically acceptable carrier. In some embodiments, the pharmaceutical composition is formulated for intravenous administration, intracerebral administration, or intrathecal administration.

In the sixth aspect, provided is a method for preventing or treating Alzheimer's disease, comprising administering to a subject in need thereof the modified TPK polypeptide, polynucleotide, rAAV, recombinant lentivirus or pharmaceutical composition according to the present disclosure. In some embodiments, the modified TPK polypeptide, polynucleotide, rAAV, recombinant lentivirus or pharmaceutical composition is administered intravenously, intracerebrally, or intrathecally.

Provided is also use of the modified TPK polypeptide, polynucleotide, rAAV, recombinant lentivirus or pharmaceutical composition according to the present disclosure for the manufacture of a medicament for preventing or treating Alzheimer's disease. In some embodiments, the medicament is administered intravenously, intracerebrally, or intrathecally.

Further provided is the modified TPK polypeptide, polynucleotide, rAAV, recombinant lentivirus or pharmaceutical composition according to the present disclosure for use in preventing or treating Alzheimer's disease. In some embodiments, the modified TPK polypeptide, polynucleotide, rAAV, recombinant lentivirus or pharmaceutical composition is administered intravenously, intracerebrally, or intrathecally.

### Brief Description of the Drawings

Fig. 1 shows the map of the plasmid comprising the genome of AAV-TPK (Fig. 1A) and the map of the plasmid comprising the genome of TPK lentivirus (Fig. 1B).
Fig. 2 shows the image of neuronal cells infected with lentivirus by fluorescent microscopy.
Fig. 3 shows the TPK expression (Fig. 3A, parental representing cells without viral infection), TDP content (Fig. 3B), and TM content (Fig. 3C) in neuronal cells infected with lentivirus.
Fig. 4 shows the body weight (left panel and middle panel) and food intake (right panel) of mice injected with rAAV via tail vein.
Fig. 5 shows the TDP content (left panel) and TM content (right panel) in blood of mice injected with rAAV via tail vein.
Fig. 6 shows the images by immunofluorescence of slices in various regions of brain of mice injected with rAAV via tail vein.
Fig. 7 shows the TPK expression (left panel) and the contents of TDP and TM (right panel) in brain of mice injected with rAAV via tail vein.
Fig. 8 shows the TDP content (left panel) and TM content (right panel) in liver of mice injected with rAAV via tail vein.
Fig. 9 shows the body weight (left panel and middle panel) and food intake (right panel) of mice injected with rAAV in lateral ventricle.
Fig. 10 shows the TDP content (left panel) and TM content (right panel) in blood of mice injected with rAAV in lateral ventricle.
Fig. 11 shows images by immunofluorescence of slices in various regions of brain of mice injected with rAAV in lateral ventricle.
Fig. 12 shows the expression of TPK (left panel) and the contents of TDP and TM (right panel) in brain of mice injected with rAAV in lateral ventricle.
Fig. 13 shows the TDP content (left panel) and TM content (right panel) in liver of mice injected with rAAV in lateral ventricle.
Fig. 14 shows the weight changes of mice developed from embryos injected with rAAV in lateral ventricle in 2 weeks after the weaning, i.e., 21 days after birth.
Fig. 15 shows TDP content (left panel) and TM content (right panel) in blood of mice developed from embryos injected with rAAV in lateral ventricle 21 days after birth.
Fig. 16 shows images by immunofluorescence of slices in various regions of brain of mice developed from embryos injected with rAAV in lateral ventricle 21 days after birth.
Fig. 17 shows the TPK expression (left panel) and contents of TDP and TM (right panel) in brain of mice developed from embryos injected with rAAV in lateral ventricle 21 days after birth.
Fig. 18 shows the TDP content (left panel) and TM content (right panel) in liver of mice developed from embryos injected with rAAV in lateral ventricle 21 days after birth.
Fig. 19 shows the weight changes in 2 weeks after weaning, i.e., 21 days after the injection of rAAV into the lateral ventricle of neonatal mice.
Fig. 20 shows the TDP content (left panel) and TM content (right panel) in blood 21 days after the injection of rAAV into the lateral ventricle of neonatal mice.
Fig. 21 shows the images by immunofluorescence of slices in various regions of brain 21 days after the injection of rAAV into the lateral ventricle of neonatal mice.
Fig. 22 shows the TPK expression (left panel) and contents of TDP and TM (right panel) in brain 21 days after the injection of rAAV into the lateral ventricle of neonatal mice.
Fig. 23 shows the weight changes in 2 weeks after the weaning, i.e., 21 days after the injection of rAAV into the hippocampus of neonatal mice.
Fig. 24 shows TDP content (left panel) and TM content (right panel) in blood 21 days after the injection of rAAV into the hippocampus of neonatal mice.
Fig. 25 shows the images by immunofluorescence of slices in various regions of brain 21 days after the injection of rAAV into the hippocampus of neonatal mice.
Fig. 26 shows the TPK expression in brain 21 days after the injection of rAAV into the hippocampus of neonatal mice.
Fig. 27 shows the TDP content (left panel) and TM content (right panel) in liver 21 days after the injection of rAAV into the hippocampus of neonatal mice.
Fig. 28 shows the comparison in activity of the modified human TPK with wild-type human TPK and mouse TPK.
Fig. 29 shows the comparison in activity of the modified mouse TPK with wild-type mouse TPK.
Fig. 30 shows the Western blot results of TPK protein in brain tissues.
Fig. 31 shows the HPLC results of TDP content in brain tissues.
Fig. 32 shows the curves of body weight (A), food intake (B), and blood glucose (C) of mice injected with AAV-hTPK.
Fig. 33 shows the Western blot results of TPK protein in brain tissues and liver of APP/PS1 mice injected with AAV-TPK.
Fig. 34 shows the contents of TDP and TM in brain tissues, liver, and blood of APP/PS1 mice injected with AAV-TPK.
Fig. 35 shows the Western blot results of p-GSK3β and GSK3β in brain tissues of APP/PS1 mice injected with AAV-TPK in lateral ventricle.
Fig. 36 shows the p-GSK3β/GSK3β ratio in brain tissues of APP/PS1 mice injected with AAV-TPK in lateral ventricle.

### Detailed Description of the Invention

### I. Definition

Unless otherwise defined, all technical and scientific terms used herein have the same meanings as those commonly understood by those skilled in the field to which the present disclosure belongs. Although any methods and materials similar or equivalent to those described herein may also be used in practice and teaching of the present disclosure, preferable methods and materials will be described. All publications mentioned herein are incorporated by reference to disclose and describe the methods and/or materials cited together with the publications.

"Thiamine pyrophosphokinase" or "TPK" refers to an enzyme which catalyzes the reaction converting thiamine (TM) into thiamine diphosphate (TDP).

"AAV" is abbreviation for adeno-associated virus and may refer to the virus *per se* or its derivative. Unless otherwise required, the term covers all subtypes and naturally occurring and recombinant forms. The abbreviation "rAAV" refers to a recombinant adeno-associated virus, also known as recombinant AAV vector (or "rAAV vector"). The term "AAV" comprises type 1 AAV (AAV-1), type 2 AAV (AAV-2), type 3 AAV (AAV-3), type 4 AAV (AAV-4), type 5AAV(AAV-5), type 6 AAV(AAV-6), type 7 AAV(AAV-7), type 8 AAV(AAV-8), avian AAV, cattle AAV, canine AAV, horse AAV, primate AAV, non-primate AAV and sheep AAV. "Primate AAV" refers to an AAV infecting primates, "non-primate AAV" refers to an AAV infecting non-primate mammals, "cattle AAV" refers to an AAV infecting cattle mammals, or the like.

The genome sequences of different subtypes of AAV as well as the sequences of natural terminal repeat (TR), Rep protein, and capsid subunits are known in the art. Such sequences can be found in literature or public databases such as gene libraries. See, for example, Genbank Access Numbers NC_002077 (AAV-1), AF063497 (AAV-1), NC_001401 (AAV-2), AF043303 (AAV-2), NC_001729(AAV-3), NC_001829 (AAV-4), U89790 (AAV-4), NC_006152 (AAV-5), AF513851 (AAV- 7), AF513852(AAV-8) 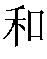 NC_006261 (AAV-8); of which the disclosures are incorporated herein by reference, for teaching nucleic acid and amino acid sequences of AAV

As used herein, "rAAV vector" refers to an AAV vector which contains a polynucleotide sequence from non-AAV source (i.e., a polynucleotide heterologous from AAV), usually an AAV vector for target sequence for cellular genetic transformation. Generally, the heterologous polynucleotide is flanked by at least one and typically two AAV reverse terminal repeat sequences (ITR). The term rAAV vector encompasses an rAAV vector particle and an rAAV vector plasmid. The rAAV vector may be single-stranded (ssAAV) or self-complementary (scAAV).

"Packaging" guides a series of intracellular events leading to the assembly and capsidation of AAV particles.

AAV "rep" and "cap" genes refer to polynucleotide sequences encoding the replication and capsidation proteins of adeno-associated virus. AAV rep and cap are also known as AAV packaging genes.

"Helper virus" of AAV refers to a virus which allows the replication and packaging of AAV (such as wildtype AAV) in mammalian cells. Many helper viruses of AAV are known in the field, including adenoviruses, herpesviruses and poxviruses (such as vaccinia). Although adenovirus type 5 of C subclass is the most commonly used, the adenovirus covers many different subclasses. It is known that many adenoviruses of human, non-human mammalian and avian origin can be obtained from depository institutions such as ATCC. Viruses from Herpesviridae comprise, for example, herpes simplex virus (HSV) and Epstein-Barr viruses (EBV), as well as cytomegalovirus (CMV) and pseudorabies virus (PRV); which can be obtained from depository institutions, such as ATCC.

"Helper virus function" refers to function encoded in the genome of helper virus which allows AAV replication and packaging (along with other requirements for replication and packaging described herein). As mentioned herein, the "helper virus function" may be provide in various ways, including by providing a helper virus or by providing (for example) a trans polynucleotide sequence which encodes essential functions for production cells. For example, a plasmid containing a nucleotide sequence encoding one or more adenovirus protein(s) (also known as auxiliary plasmid) or other expression vector, along with rAAV vector, is transfected into production cells.

Lentivirus is a viral vector derived from HIV-1. "Recombinant lentivirus vector" or "recombinant lentivirus vector particle" refers to recombinant virus particle and recombinant virus like particle produced in host or production cells after plasmid vector transfection, wherein the plasmid vector is composed of a transfer vector, an envelope vector encoding selected envelope protein, and a packaging vector which provides lentivirus proteins (such as lentivirus GAG and POL proteins, especially mutated POL protein to prevent integration) in a trans manner according to well-known methods in the art.

Virus-like particles are produced by incomplete assembly of proteins present for the capsidation of the genome of the recombinant lentivirus, and the method as used cannot form true viral particles.

The term "polynucleotide" refers to nucleotide polymerization form in any length, including deoxyribonucleotides or ribonucleotides or analogues thereof. The polynucleotide may comprise modified nucleotides, such as methylated nucleotides and nucleotide analogues, and may be interrupted by non-nucleotide components. If present, modifications to nucleotide structure can be applied before or after polymer assembly. As used herein, the term polynucleotide may refer to alternating double-stranded and single-stranded molecules. Unless otherwise specified or required, any embodiment of the present disclosure herein as polynucleotide encompasses each of the double-stranded form and two complementary single-stranded forms known or predicted to constitute the double stranded form.

Nucleic acid hybridization reactions may be conducted under different "stringent" conditions. The conditions for enhancing stringency of hybridization reactions are well-known and publicly disclosed in the field. See, for example, Sambrook et. al., Molecular Cloning, A Laboratory Manual, 2nd edition, Cold Spring Harbor Laboratory Press, Cold Spring Harbor, N.Y., 1989, which is incorporated herein by reference.

A polynucleotide or polypeptide having a certain percentage of "sequence identity" with another polynucleotide or polypeptide refers to the percentage of bases or amino acids which are identical when comparing two sequences in alignment. There are many different methods to determine sequence similarity. To determine sequence identity, methods and computer programs which can be used to align sequences comprise for example, BLAST, which can be obtained in ncbi.nlm.nih.gov/BLAST/, and FASTA, which can be obtained in the Genetics Computing Group (GCG) package from Madison, Wisconsin, USA, a wholly owned subsidiary of Oxford Molecular Group, Inc.

"Gene" refers to polynucleotides which contain at least one open reading frame capable of encoding a specific protein after transcription and translation.

If applied to a polynucleotide, "recombination" means that the polynucleotide is the product of different combinations of cloning, restriction, or ligation steps, and other procedures which produce a construct different from those found in nature. A recombinant virus is a viral particle comprising a recombinant polynucleotide. The term comprises pro-polynucleotide construct or a duplicate of pro-virus construct progeny.

"Control element" or "control sequence" is a nucleotide sequence involved in molecular interactions which contribute to functional regulations of the polynucleotide, including replication, duplication, transcription, splicing, translation, or degradation of the polynucleotide. Regulation may affect frequency, speed, or specificity of the process, and may actually be either enhancing or inhibiting. Control elements known in the field comprise, for example, transcriptional regulatory sequences, such as promoter and enhancer. The promoter is a DNA region which can bind to RNA polymerase under certain conditions and initiate transcription of the coding region typically downstream (3' direction) of the promoter.

"Operatively linked" refers to parallelism of genetic elements, wherein the elements show relationship allowing them to operate in an expected manner. For example, if a promoter assists in initiating transcription of a coding sequence, the promoter is operably linked to the coding region. As long as such functional relationship is maintained, there may be residues inserted between the promoter and the coding region.

"Expression vector" is a vector containing a region encoding the target polypeptide and used to achieve protein expression in a predetermined target cell. The expression vector further comprises a control element which is operatively linked to the coding region to promote protein expression in a target. The combination of a control element and gene which can be operably linked for expression is sometimes referred to as "expression cassette", and many expression cassettes are known in the field and can be obtained or easily constructed from components available in the field.

"Heterologous" refers to an entity originating from a different genotype as compared to another entity. For example, a polynucleotide introduced into a plasmid or vector from different species through genetic engineering techniques is a heterologous polynucleotide. A promoter taken from its natural encoding sequence and operatively linked to an encoding sequence which is not found as naturally linked thereto is a heterologous promoter. Therefore, for example, an rAAV containing a heterologous nucleic acid encoding a heterologous gene product is an rAAV containing a nucleic acid which is generally not included in a naturally occurring wildtype AAV, and the encoded heterologous gene product is generally not a naturally occurring wildtype AAV encoded gene product.

The terms "polypeptide", "peptide", and "protein" can be interchanged used herein to refer to an amino acid polymer in any length. The terms also encompass a modified amino acid polymer; for example, disulfide bond formation, glycosylation, lipoylation, phosphorylation, or binding to a labeled component.

The modification also comprises that to a polypeptide sequence, including but not limited to substitution, deletion, insertion, and/or addition of one or more amino acids.

The term "conservative substitution", also known as substitution with "homologous" amino acid residue, refers to an amino acid residue which is replaced with another amino acid with a similar side chain; for example, amino acids with alkaline side chains (such as lysine, arginine, and histidine), amino acids with acidic side chains (such as aspartic acid and glutamic acid), amino acids with uncharged, polar side chains (such as glycine, asparagine, glutamine, serine, threonine, tyrosine, and cysteine), amino acids with non-polar side chains (such as alanine, valine, leucine, isoleucine, proline, phenylalanine, methionine, and tryptophan), amino acids with β-branched side chains (such as threonine, valine, and isoleucine), and amino acids with aromatic side chains (such as tyrosine, phenylalanine, tryptophan, and histidine).

Conservative amino acid substitution usually has the least influence on activity of the resultant protein. Such substitutions will be described hereafter. Conservative substitution is substitution of an amino acid with another amino acid similar in size, hydrophobicity, charge, polarity, spatial characteristic, aromaticity, or the like. When it is desirable to finely regulate the properties of a protein, the substitution is usually conservative.

As used herein, "homologous" amino acid residues refer to amino acid residues with similar chemical properties, involving hydrophobicity, charge, polarity, spatial characteristic, aromaticity, or the like. Examples of amino acids which are homologous to each other comprise positively charged lysine, arginine, histidine; negatively charged glutamic acid, aspartic acid; hydrophobic glycine, alanine, valine, leucine, isoleucine, proline, phenylalanine; polar serine, threonine, cysteine, methionine, tryptophan, tyrosine, asparagine, glutamine; aromatic phenylalanine, tyrosine, tryptophan; serine and threonine with chemically similar side chain groups, or glutamine and asparagine, or leucine and isoleucine.

Examples of conserved amino acid substitutions in proteins comprise: Ser for Ala, Lys for Arg, Gln or His for Asn, Glu for Asp, Ser for Cys, Asn for Gln, Asp for Glu, Pro for Gly, Asn or Gln for His, Leu or Val for Ile, Ile or Val for Leu, Arg or Gln for Lys, Leu or Ile for Met, Met, Leu or Tyr for Phe, Thr for Ser, Ser for Thr, Tyr for Trp, Trp or Phe for Tyr, and Ile or Leu for Val.

"Isolated" plasmid, nucleic acid, vector, virus, viral body, host cell, or other substance refer to a preparation which does not contain at least some other components of the substance or similar substance which may be present as naturally occurring or originally prepared. Therefore, for example, purification technique may be used to prepare isolated substance for enrichment from the original mixture.

As used herein, the term "treatment" refers to obtaining desirable pharmacological and/or physiological effects. The effects may be preventive in completely or partially preventing a disease or its symptom, and/or therapeutic in partially or completely curing a disease and/or a side effect caused by the disease. As used herein, "treatment" comprises any treatment for a disease in a mammal (especially human), and comprises: (a) preventing the disease from occurring in a subject who is susceptible to or at risk of the disease but has not yet been diagnosed with the disease; (b) inhibiting the disease, that is, preventing its development; and (c) alleviating the disease, that is, cause recession of the disease.

The terms "individual", "host", "subject", and "patient" can be used interchangeably herein and refer to mammals, including but not limited to human and non-human primates, including ape and human; mammalian sport animals (such as horse); mammalian livestock (such as sheep, goat, or the like); mammalian pets (dog, cat, or the like); and rodents (such as mouse, rat, or the like).

### II. Modified TPK polypeptide

The present inventors find that catalytic activity of converting TM to TDP thiamine of mouse pyrophosphokinase (mTPK) is higher than that of human TPK (hTPK). The present inventors modify hTPK and mTPK sequences to enhance catalytic activity of converting TM to TDP. For example, the surface charge properties of hTPK are modified according to murine origin, that is, when the charge properties of residue Xh in hTPK is different from residue Xm at the aligned position in mTPK, the residue Xh in hTPK is substituted with Xm.

Accordingly, provided is a modified thiamine pyrophosphokinase (TPK) polypeptide comprising the substitution of amino acid at one or more positions selected from the group consisting of positions 13, 30, 31, 37, 85, 129, 158, 181, 11 and 35, as compared to an initial TPK polypeptide, wherein the modified TPK polypeptide has an improved catalytic activity of converting TM to TDP as compared to the initial TPK polypeptide, and wherein the positions are numbered by reference to SEQ ID NO: 1 or 8.

Preferably, the amino acid at position 13 is substituted with P. Preferably, the amino acid at position 30 is substituted with A. Preferably, the amino acid at position 31 is substituted with R. Preferably, the amino acid at position 37 is substituted with K. Preferably, the amino acid at position 85 is substituted with K. Preferably, the amino acid at position 129 is substituted with G. Preferably, the amino acid at position 158 is substituted with K. Preferably, the amino acid at position 181 is substituted with S. Preferably, the amino acid at position 11 is substituted with W. Preferably, the amino acid at position 35 is substituted with W.

As used herein, the TPK polypeptide, based on which amino acids are modified, is designated as the initial TPK polypeptide. The initial TPK polypeptide may be wildtype TPK polypeptide, or a variant thereof. For example, if the modifications are conducted on the polypeptide of SEQ ID NO: 1, the polypeptide of SEQ ID NO: 1 is the "initial TPK polypeptide" for the modified TPK polypeptide; and if the modifications are conducted on a variant of the polypeptide of SEQ ID NO: 1, the variant polypeptide is the "initial TPK polypeptide" for the modified TPK polypeptide.

In some embodiments, the initial TPK polypeptide is a wildtype TPK polypeptide, for example, human TPK polypeptide and mouse TPK polypeptide.

In some embodiments, the initial TPK polypeptide is human TPK polypeptide, and wherein the modified TPK polypeptide comprises the substitution of amino acid at one or more positions selected from the group consisting of positions 13, 30, 31, 37, 85, 129, 158, 181, 11 and 35. In some embodiments, the initial TPK polypeptide is human TPK polypeptide, and wherein the modified TPK polypeptide comprises the substitution of amino acid at one or more positions selected from the group consisting of positions 13, 30, 31, 37, 85, 129, 158 and 181. Preferably, the initial TPK polypeptide is human TPK polypeptide, and wherein the modified TPK polypeptide comprises the substitution of amino acid at positions 13, 30, 31, 37, 85, 129, 158 and 181.

In some embodiments, the initial TPK polypeptide is mouse TPK polypeptide, and the modified TPK polypeptide comprises the substitution of amino acid at positions 11 and/or 35.

In some embodiments, the initial TPK polypeptide comprises an amino acid sequence of SEQ ID NO: 8, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 8, wherein the modified TPK polypeptide comprises the substitution of amino acid at one or more positions selected from the group consisting of positions 13, 30, 31, 37, 85, 129, 158, 181, 11 and 35, for example the substitution of amino acid at positions 13, 30, 31, 37, 85, 129, 158 and 181, positions 13, 30, 31, 37, 85, 129, 158, 181 and 11, positions 13, 30, 31, 37, 85, 129, 158, 181 and 35, or positions 13, 30, 31, 37, 85, 129, 158, 181, 11 and 35, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises the substitution of amino acid at positions 11W and/or 35W, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises the substitution of amino acid at position 11W, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises an amino acid substitution of amino acid substitution at position 35W, as compared to the initial TPK polypeptide.

In some embodiments, the modified TPK polypeptide comprises the following substitution of amino acid or combination of substitutions of amino acids:
11W;
35W;
13P-30A-31R-37K-85K-129G-158K-181S;
13P-30A-31R-37K-85K-129G-158K-181S-11W;
13P-30A-31R-37K-85K-129G-158K-181S-35W; or
13P-30A-31R-37K-85K-129G-158K-181S-11W-35W.

In some embodiments, the modified TPK polypeptide comprises an amino acid sequence of SEQ ID NO: 9, 10 or 11, or an amino acid sequence having at least 65%, preferably at least 70%, 75% or 80%, more preferably at least 85%, 90% or 95%, particularly preferably at least 96%, 97%, 98% or 99% sequence identity to SEQ ID NO: 9, 10 or 11. For example, as compared to SEQ ID NO: 9, 10 or 11, the modified TPK polypeptide comprises one or more substitutions of amino acids.

In some embodiments, the modified TPK polypeptide comprises the substitution, preferably conservative substitution, of 1, 2, 3, 4, 5, 6, 7, 8, 9, 10 or more amino acids at positions other than positions 13, 30, 31, 37, 85, 129, 158 and 181, and preferably 241, as compared to the amino acid sequence of SEQ ID NO: 9.

In some embodiments, the modified TPK polypeptide comprises the substitution, preferably conservative substitution, of 1, 2, 3, 4, 5, 6, 7, 8, 9, 10 or more amino acids at positions other than positions 11 and 241, and preferably 13, 30, 31, 37, 85, 129, 158 and 181, as compared to the amino acid sequence of SEQ ID NO: 10.

In some embodiments, the modified TPK polypeptide comprises the substitution, preferably conservative substitution, of 1, 2, 3, 4, 5, 6, 7, 8, 9, 10 or more amino acids at positions other than positions 35 and 241, and preferably 13, 30, 31, 37, 85, 129, 158 and 181, as compared to the amino acid sequence of SEQ ID NO: 11.

In some embodiments, the modified TPK polypeptide has catalytic activity of converting TM to TDP, which is at least 110%, 120%, 130%, 140%, 150%, 200%, 250%, 300% or more of that of the initial TPK polypeptide.

### III. Isolated polynucleotide and expression cassette

Provided is an isolated polynucleotide encoding a thiamine pyrophosphokinase (TPK).

The TPK can be a TPK derived from human or non-human animal, such as non-human mammals. Examples of TPK derived from non-human mammals include but not limited to those from non-human primates such as monkey, horse, cow, sheep, goat, pig, dog, cat, mouse, and rat.

In some embodiments, the TPK is a wildtype TPK. In some embodiments, the TPK is mouse TPK or human TPK.

In some embodiments, the TPK comprises an amino acid sequence of SEQ ID NO: 1. In some embodiments, the polynucleotide encoding the TPK comprises a nucleotide sequence of SEQ ID NO: 2. In some embodiments, the TPK consists of an amino acid sequence of SEQ ID NO: 1. In some embodiments, the polynucleotide encoding the TPK consists of a nucleotide sequence of SEQ ID NO: 2.

In some embodiments, the TPK is a modified TPK comprising the substitution, insertion, deletion and/or addition of one or more amino acids, as compared to the initial TPK, wherein the activity of the modified TPK is at least 10%, 20%, 30%, 40%, 50%, 60%, 70%, 80%, 90%, 100%, 110%, 120%, 130%, 140%, 150%, 200%, 250%, 300% or more of that of the initial TPK. In some embodiments, the modified TPK comprises the substitution, insertion, deletion and/or addition of 1, 2, 3, 4, 5, 6, 7, 8, 9, 10 or more amino acids, as compared to the initial TPK. In some embodiments, the modified TPK has at least 80%, 90%, 95%, 96%, 97%, 98%, 99% or more identity to the initial TPK.

The initial TPK can be a wildtype TPK or a modified TPK. In some embodiments, the initial TPK is a wildtype mouse TPK or a wildtype human TPK.

In some embodiments, the initial TPK comprises an amino acid sequence of SEQ ID NO: 1. In some embodiments, the initial TPK consists of an amino acid sequence of SEQ ID NO: 1.

In some embodiments, as compared to the initial TPK polypeptide, the modified TPK polypeptide comprises the substitution of amino acid at one or more positions selected from the group consisting of positions 13, 30, 31, 37, 85, 129, 158, 181, 11 and 35, as compared to an initial TPK polypeptide, wherein the modified TPK polypeptide has an improved catalytic activity of converting TM to TDP as compared to the initial TPK polypeptide, and wherein the positions are numbered by reference to SEQ ID NO: 1 or 8.

Preferably, the amino acid at position 13 is substituted with P. Preferably, the amino acid at position 30 is substituted with A. Preferably, the amino acid at position 31 is substituted with R. Preferably, the amino acid at position 37 is substituted with K. Preferably, the amino acid at position 85 is substituted with K. Preferably, the amino acid at position 129 is substituted with G. Preferably, the amino acid at position 158 is substituted with K. Preferably, the amino acid at position 181 is substituted with S. Preferably, the amino acid at position 11 is substituted with W. Preferably, the amino acid at position 35 is substituted with W.

In some embodiments, the initial TPK polypeptide is a wildtype TPK polypeptide, for example, human TPK polypeptide and mouse TPK polypeptide.

In some embodiments, the initial TPK polypeptide is human TPK polypeptide, and wherein the modified TPK polypeptide comprises the substitution of amino acid at one or more positions selected from the group consisting of positions 13, 30, 31, 37, 85, 129, 158, 181, 11 and 35. In some embodiments, the initial TPK polypeptide is human TPK polypeptide, and wherein the modified TPK polypeptide comprises the substitution of amino acid at one or more positions selected from the group consisting of positions 13, 30, 31, 37, 85, 129, 158 and 181. Preferably, the initial TPK polypeptide is human TPK polypeptide, and wherein the modified TPK polypeptide comprises the substitution of amino acid at positions 13, 30, 31, 37, 85, 129, 158 and 181.

In some embodiments, the initial TPK polypeptide is mouse TPK polypeptide, and the modified TPK polypeptide comprises the substitution of amino acid at positions 11 and/or 35.

In some embodiments, the initial TPK polypeptide comprises an amino acid sequence of SEQ ID NO: 8, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 8, wherein the modified TPK polypeptide comprises the substitution of amino acid at one or more positions selected from the group consisting of positions 13, 30, 31, 37, 85, 129, 158, 181, 11 and 35, for example the substitution of amino acid at positions 13, 30, 31, 37, 85, 129, 158 and 181, positions 13, 30, 31, 37, 85, 129, 158, 181 and 11, positions 13, 30, 31, 37, 85, 129, 158, 181 and 35, or positions 13, 30, 31, 37, 85, 129, 158, 181, 11 and 35, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises the substitution of amino acid at positions 11W and/or 35W, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises the substitution of amino acid at position 11W, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises an amino acid substitution of amino acid substitution at position 35W, as compared to the initial TPK polypeptide.

In some embodiments, the modified TPK polypeptide comprises the substitution of amino acid or the combination of substitutions of amino acids:
11W;
35W;
13P-30A-31R-37K-85K-129G-158K-181S;
13P-30A-31R-37K-85K-129G-158K-181S-11W;
13P-30A-31R-37K-85K-129G-158K-181S-35W; or
13P-30A-31R-37K-85K-129G-158K-181S-11W-35W.

In some embodiments, the modified TPK polypeptide comprises an amino acid sequence of SEQ ID NO: 9, 10 or 11, or an amino acid sequence having at least 65%, preferably at least 70%, 75% or 80%, more preferably at least 85%, 90% or 95%, particularly preferably at least 96%, 97%, 98% or 99% sequence identity to SEQ ID NO: 9, 10 or 11. For example, as compared to SEQ ID NO: 9, 10 or 11, the modified TPK polypeptide comprises one or more substitutions of amino acids.

In some embodiments, the modified TPK polypeptide comprises the substitution, preferably conservative substitution, of 1, 2, 3, 4, 5, 6, 7, 8, 9, 10 or more amino acids at positions other than positions 13, 30, 31, 37, 85, 129, 158 and 181, and preferably 241, as compared to the amino acid sequence of SEQ ID NO: 9.

In some embodiments, the modified TPK polypeptide comprises the substitution, preferably conservative substitution, of 1, 2, 3, 4, 5, 6, 7, 8, 9, 10 or more amino acids at positions other than positions 11 and 241, and preferably 13, 30, 31, 37, 85, 129, 158 and 181, as compared to the amino acid sequence of SEQ ID NO: 10.

In some embodiments, the modified TPK polypeptide comprises the substitution, preferably conservative substitution, of 1, 2, 3, 4, 5, 6, 7, 8, 9, 10 or more amino acids at positions other than positions 35 and 241, and preferably 13, 30, 31, 37, 85, 129, 158 and 181, as compared to the amino acid sequence of SEQ ID NO: 11.

In some embodiments, the modified TPK polypeptide has catalytic activity of converting TM to TDP, which is at least 110%, 120%, 130%, 140%, 150%, 200%, 250%, 300% or more of that of the initial TPK polypeptide.

In some embodiments, the polynucleotide comprises a nucleotide sequence of SEQ ID NO: 15, 16 or 17, or a nucleotide sequence having at least 70%, 75%, 80%, 85%, 90%, 95%, 96%, 97%, 98% or 99% sequence identity to SEQ ID NO: 15, 16 or 17.

In some embodiments, the polynucleotide comprises a nucleotide sequence of SEQ ID NO: 15, or a nucleotide sequence having at least 70%, 75%, 80%, 85%, 90%, 95%, 96%, 97%, 98% or 99% sequence identity to SEQ ID NO: 15. In some embodiments, the polynucleotide comprises a nucleotide sequence of SEQ ID NO: 15, or a nucleotide sequence having at least 70%, 75%, 80%, 85%, 90%, 95%, 96%, 97%, 98% or 99% sequence identity to SEQ ID NO: 15; and the nucleotide sequence encodes an amino acid sequence comprising the substitution, preferably conservative substitution, of 1, 2, 3, 4, 5, 6, 7, 8, 9, 10 or more amino acids at positions other than positions other than positions 13, 30, 31, 37, 85, 129, 158 and 181, and preferably 241 as compared to SEQ ID NO: 9.

In some embodiments, the polynucleotide comprises a nucleotide sequence of SEQ ID NO: 16, or a nucleotide sequence having at least 70%, 75%, 80%, 85%, 90%, 95%, 96%, 97%, 98% or 99% sequence identity to SEQ ID NO: 16. In some embodiments, the polynucleotide comprises a nucleotide sequence of SEQ ID NO: 16, or a nucleotide sequence having at least 70%, 75%, 80%, 85%, 90%, 95%, 96%, 97%, 98% or 99% sequence identity to SEQ ID NO: 16; and the nucleotide sequence encodes an amino acid sequence comprising the substitution, preferably conservative substitution, of 1, 2, 3, 4, 5, 6, 7, 8, 9, 10 or more amino acids at positions other than positions 11 and 241, and preferably 13, 30, 31, 37, 85, 129, 158 and 181, as compared to SEQ ID NO: 10.

In some embodiments, the polynucleotide comprises a nucleotide sequence of SEQ ID NO: 17, or a nucleotide sequence having at least 70%, 75%, 80%, 85%, 90%, 95%, 96%, 97%, 98% or 99% sequence identity to SEQ ID NO: 17. In some embodiments, the polynucleotide comprises a nucleotide sequence of SEQ ID NO: 17, or a nucleotide sequence having at least 70%, 75%, 80%, 85%, 90%, 95%, 96%, 97%, 98% or 99% sequence identity to SEQ ID NO: 17; and the nucleotide sequence encodes an amino acid sequence comprising the substitution, preferably conservative substitution, of 1, 2, 3, 4, 5, 6, 7, 8, 9, 10 or more amino acids at positions other than positions 35 and 241, and preferably 13, 30, 31, 37, 85, 129, 158 and 181, as compared to SEQ ID NO: 11.

In some embodiments, the polynucleotide is DNA or RNA, for example mRNA.

Provided is also an expression cassette, comprising the polynucleotide according to the present disclosure, operatively linked to a promoter.

In some embodiments, the promoter is a neuron-specific promoter. In some embodiments, the promoter comprises a nucleotide sequence of SEQ ID NO: 3. In some embodiments, the promoter consists of a nucleotide sequence of SEQ ID NO: 3. In some embodiments, the promoter comprises a nucleotide sequence having at least 70%, 80%, 90%, 95%, 96%, 97%, 98%, 99% or more sequence identity to SEQ ID NO: 3. In some embodiments, the promoter consists of a nucleotide sequence having at least 70%, 80%, 90%, 95%, 96%, 97%, 98%, 99% or more sequence identity to SEQ ID NO: 3.

In some embodiments, the promoter is a eukaryotic strong promoter, for example, cytomegalovirus (CMV) promoter. In some embodiments, the promoter comprises a nucleotide sequence of SEQ ID NO: 7. In some embodiments, the promoter consists of a nucleotide sequence of SEQ ID NO: 7. In some embodiments, the promoter comprises a nucleotide sequence having at least 70%, 80%, 90%, 95%, 96%, 97%, 98%, 99% or more sequence identity to SEQ ID NO: 7. In some embodiments, the promoter consists of a nucleotide sequence having at least 70%, 80%, 90%, 95%, 96%, 97%, 98%, 99% or more sequence identity to SEQ ID NO: 7.

The expression cassette can comprise an additional control element, such as an enhancer, an intron, an mRNA stabilizing sequence and a polyadenylation signal sequence.

In some embodiments, the expression cassette further comprises an mRNA stabilizing sequence, for example a WPRE element. In some embodiments, the WPRE element comprises a nucleotide sequence of SEQ ID NO: 4. In some embodiments, the WPRE element consists of a nucleotide sequence of SEQ ID NO: 4. In some embodiments, the WPRE element comprises a nucleotide sequence having at least 70%, 80%, 90%, 95%, 96%, 97%, 98%, 99% or more identity to SEQ ID NO: 4. In some embodiments, the WPRE element consists of a nucleotide sequence having at least 70%, 80%, 90%, 95%, 96%, 97%, 98%, 99% or more identity to SEQ ID NO: 4.

In some embodiments, the WPRE element is downstream of the polynucleotide encoding TPK.

In some embodiments, the expression cassette further comprises a polyadenylation signal sequence, for example, but not limited to polyadenylation signal sequence of human growth factor gene and polyadenylation signal sequence of rabbit globulin gene. In some embodiments, the expression cassette further comprises polyadenylation signal sequence of human growth factor gene. In some embodiments, the polyadenylation signal sequence comprises a nucleotide sequence of SEQ ID NO: 5. In some embodiments, the polyadenylation signal sequence consists of a nucleotide sequence of SEQ ID NO: 5. In some embodiments, the polyadenylation signal sequence comprises a nucleotide sequence having at least 70%, 80%, 90%, 95%, 96%, 97%, 98%, 99% or more identity to SEQ ID NO: 5. In some embodiments, the polyadenylation signal sequence consists of a nucleotide sequence having at least 70%, 80%, 90%, 95%, 96%, 97%, 98%, 99% or more identity to SEQ ID NO: 5.

In some embodiments, the polyadenylation signal sequence is downstream of the WPRE element.

The expression cassette may be a part of the genome of the rAAV. Accordingly, expression cassette is flanked by ITR of the AAV Non-limiting examples of the ITR comprise those derived from AAV-1, AAV-2, AAV-3, AAV-4, AAV-5, AAV-6, AAV-7, AAV-8, AAV-9, poultry AAV, cattle AAV, canine AAV, horse AAV, primate AAV, non-primate AAV and sheep AAV

In some embodiments, the ITR is ITR of AAV-2. In some embodiments, the 5' ITR is identical to the 3' ITR. In some embodiments, the 5' ITR is different from the 3' ITR.

In some embodiments, the 5' ITR and the 3' ITR are both ITR130, for example, comprising a nucleotide sequence of SEQ ID NO: 6. In some embodiments, the 5' ITR and the 3' ITR consists of a nucleotide sequence of SEQ ID NO: 6, respectively. In some embodiments, the 5' ITR and the 3' ITR comprise a nucleotide sequence having at least 70%, 80%, 90%, 95%, 96%, 97%, 98%, 99% or more identity to SEQ ID NO: 6, respectively. In some embodiments, the 5' ITR and the 3' ITR consist of a nucleotide sequence having at least 70%, 80%, 90%, 95%, 96%, 97%, 98%, 99% or more identity to SEQ ID NO: 6, respectively.

In some embodiments, the expression cassette comprises, from 5' to 3', SEQ ID NO: 3, SEQ ID NO: 2, SEQ ID NO: 4, SEQ ID NO: 5. In some embodiments, the genome of the rAAV comprises, from 5' to 3', SEQ ID NO: 6, SEQ ID NO: 3, SEQ ID NO: 2, SEQ ID NO: 4, SEQ ID NO: 5, SEQ ID NO: 6.

In some embodiments, the expression cassette comprises, from 5' to 3', SEQ ID NO: 3, SEQ ID NO: 14, SEQ ID NO: 4, SEQ ID NO: 5. In some embodiments, the genome of the rAAV comprises, from 5' to 3', SEQ ID NO: 6, SEQ ID NO: 3, SEQ ID NO: 14, SEQ ID NO: 4, SEQ ID NO: 5, SEQ ID NO: 6.

In some embodiments, the expression cassette comprises, from 5' to 3', SEQ ID NO: 3, SEQ ID NO: 15, SEQ ID NO: 4, SEQ ID NO: 5. In some embodiments, the genome of the rAAV comprises, from 5' to 3', SEQ ID NO: 6, SEQ ID NO: 3, SEQ ID NO: 15, SEQ ID NO: 4, SEQ ID NO: 5, SEQ ID NO: 6.

In some embodiments, the expression cassette comprises, from 5' to 3', SEQ ID NO: 3, SEQ ID NO: 16, SEQ ID NO: 4, SEQ ID NO: 5. In some embodiments, the genome of the rAAV comprises, from 5' to 3', SEQ ID NO: 6, SEQ ID NO: 3, SEQ ID NO: 16, SEQ ID NO: 4, SEQ ID NO: 5, SEQ ID NO: 6.

In some embodiments, the expression cassette comprises, from 5' to 3', SEQ ID NO: 3, SEQ ID NO: 17, SEQ ID NO: 4, SEQ ID NO: 5. In some embodiments, the genome of the rAAV comprises, from 5' to 3', SEQ ID NO: 6, SEQ ID NO: 3, SEQ ID NO: 17, SEQ ID NO: 4, SEQ ID NO: 5, SEQ ID NO: 6.

In some embodiments, the expression cassette comprises, from 5' to 3', SEQ ID NO: 7, SEQ ID NO: 2, SEQ ID NO: 4, SEQ ID NO: 5.

In some embodiments, the expression cassette comprises, from 5' to 3', SEQ ID NO: 7, SEQ ID NO: 14, SEQ ID NO: 4, SEQ ID NO: 5.

In some embodiments, the expression cassette comprises, from 5' to 3', SEQ ID NO: 7, SEQ ID NO: 15, SEQ ID NO: 4, SEQ ID NO: 5.

In some embodiments, the expression cassette comprises, from 5' to 3', SEQ ID NO: 7, SEQ ID NO: 16, SEQ ID NO: 4, SEQ ID NO: 5.

In some embodiments, the expression cassette comprises, from 5' to 3', SEQ ID NO: 7, SEQ ID NO: 17, SEQ ID NO: 4, SEQ ID NO: 5.

Provided is also a vector comprising the polynucleotide or expression cassette according to the present disclosure.

In some embodiments, the vector is an expression vector. In some embodiments, the vector is a eukaryotic expression vector.

In some embodiments, the vector is a vector providing the genome of the rAAV in rAAV packaging, i.e., transgenic plasmid.

In some embodiments, the vector is a vector providing the genome of lentivirus in lentivirus packaging, i.e., transgenic plasmid.

### IV Recombinant virus

Provided is a recombinant adeno-associated virus (rAAV) or recombinant lentivirus, comprising in the genome an expression cassette comprising a polynucleotide encoding TPK operatively linked to a promoter.

The TPK can be a TPK derived from human or non-human animal, such as non-human mammals. Examples of TPK derived from non-human mammals include but not limited to those from non-human primates such as monkey, horse, cow, sheep, goat, pig, dog, cat, mouse, and rat.

In some embodiments, the TPK is a wildtype TPK. In some embodiments, the TPK is mouse TPK or human TPK.

In some embodiments, the TPK comprises an amino acid sequence of SEQ ID NO: 1. In some embodiments, the polynucleotide encoding the TPK comprises a nucleotide sequence of SEQ ID NO: 2. In some embodiments, the TPK consists of an amino acid sequence of SEQ ID NO: 1. In some embodiments, the polynucleotide encoding the TPK consists of a nucleotide sequence of SEQ ID NO: 2.

In some embodiments, the TPK is a modified TPK comprising the substitution, insertion, deletion and/or addition of one or more amino acids, as compared to the initial TPK, wherein the activity of the modified TPK is at least 10%, 20%, 30%, 40%, 50%, 60%, 70%, 80%, 90%, 100%, 110%, 120%, 130%, 140%, 150%, 200%, 250%, 300% or more of that of the initial TPK. In some embodiments, the modified TPK comprises the substitution, insertion, deletion and/or addition of 1, 2, 3, 4, 5, 6, 7, 8, 9, 10 or more amino acids, as compared to the initial TPK. In some embodiments, the modified TPK has at least 80%, 90%, 95%, 96%, 97%, 98%, 99% or more identity to the initial TPK.

The initial TPK can be a wildtype TPK or a modified TPK. In some embodiments, the initial TPK is a wildtype mouse TPK or a wildtype human TPK.

In some embodiments, the initial TPK comprises an amino acid sequence of SEQ ID NO: 1. In some embodiments, the initial TPK consists of an amino acid sequence of SEQ ID NO: 1.

In some embodiments, as compared to the initial TPK polypeptide, the modified TPK polypeptide comprises the substitution of amino acid at one or more positions selected from the group consisting of positions 13, 30, 31, 37, 85, 129, 158, 181, 11 and 35, as compared to an initial TPK polypeptide, wherein the modified TPK polypeptide has an improved catalytic activity of converting TM to TDP as compared to the initial TPK polypeptide, and wherein the positions are numbered by reference to SEQ ID NO: 1 or 8.

Preferably, the amino acid at position 13 is substituted with P. Preferably, the amino acid at position 30 is substituted with A. Preferably, the amino acid at position 31 is substituted with R. Preferably, the amino acid at position 37 is substituted with K. Preferably, the amino acid at position 85 is substituted with K. Preferably, the amino acid at position 129 is substituted with G. Preferably, the amino acid at position 158 is substituted with K. Preferably, the amino acid at position 181 is substituted with S. Preferably, the amino acid at position 11 is substituted with W. Preferably, the amino acid at position 35 is substituted with W.

In some embodiments, the initial TPK polypeptide is a wildtype TPK polypeptide, for example, human TPK polypeptide and mouse TPK polypeptide.

In some embodiments, the initial TPK polypeptide is human TPK polypeptide, and wherein the modified TPK polypeptide comprises the substitution of amino acid at one or more positions selected from the group consisting of positions 13, 30, 31, 37, 85, 129, 158, 181, 11 and 35. In some embodiments, the initial TPK polypeptide is human TPK polypeptide, and wherein the modified TPK polypeptide comprises the substitution of amino acid at one or more positions selected from the group consisting of positions 13, 30, 31, 37, 85, 129, 158 and 181. Preferably, the initial TPK polypeptide is human TPK polypeptide, and wherein the modified TPK polypeptide comprises the substitution of amino acid at positions 13, 30, 31, 37, 85, 129, 158 and 181.

In some embodiments, the initial TPK polypeptide is mouse TPK polypeptide, and the modified TPK polypeptide comprises the substitution of amino acid at positions 11 and/or 35.

In some embodiments, the initial TPK polypeptide comprises an amino acid sequence of SEQ ID NO: 8, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 8, wherein the modified TPK polypeptide comprises the substitution of amino acid at one or more positions selected from the group consisting of positions 13, 30, 31, 37, 85, 129, 158, 181, 11 and 35, for example the substitution of amino acid at positions 13, 30, 31, 37, 85, 129, 158 and 181, positions 13, 30, 31, 37, 85, 129, 158, 181 and 11, positions 13, 30, 31, 37, 85, 129, 158, 181 and 35, or positions 13, 30, 31, 37, 85, 129, 158, 181, 11 and 35, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises the substitution of amino acid at positions 11W and/or 35W, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises the substitution of amino acid at position 11W, as compared to the initial TPK polypeptide.

In some embodiments, the initial TPK polypeptide comprises an amino acid sequence of SEQ ID NO: 1, or an amino acid sequence having at least 65%, preferably at least 70%, 75%or 80%, more preferably at least 85%, 90% or 95%, particularly preferably at least 96%, 97%, 98% or 99% sequence identity to SEQ ID NO: 1, wherein the modified TPK polypeptide comprises an amino acid substitution of amino acid substitution at position 35W, as compared to the initial TPK polypeptide.

In some embodiments, the modified TPK polypeptide comprises the substitution of amino acid or the combination of substitutions of amino acids:
11W;
35W;
13P-30A-31R-37K-85K-129G-158K-181S;
13P-30A-31R-37K-85K-129G-158K-181S-11W;
13P-30A-31R-37K-85K-129G-158K-181S-35W; or
13P-30A-31R-37K-85K-129G-158K-181S-11W-35W.

In some embodiments, the modified TPK polypeptide comprises an amino acid sequence of SEQ ID NO: 9, 10 or 11, or an amino acid sequence having at least 65%, preferably at least 70%, 75% or 80%, more preferably at least 85%, 90% or 95%, particularly preferably at least 96%, 97%, 98% or 99% sequence identity to SEQ ID NO: 9, 10 or 11. For example, as compared to SEQ ID NO: 9, 10 or 11, the modified TPK polypeptide comprises one or more substitutions of amino acids.

In some embodiments, the modified TPK polypeptide comprises the substitution, preferably conservative substitution, of 1, 2, 3, 4, 5, 6, 7, 8, 9, 10 or more amino acids at positions other than positions 13, 30, 31, 37, 85, 129, 158 and 181, and preferably 241, as compared to the amino acid sequence of SEQ ID NO: 9.

In some embodiments, the modified TPK polypeptide comprises the substitution, preferably conservative substitution, of 1, 2, 3, 4, 5, 6, 7, 8, 9, 10 or more amino acids at positions other than positions 11 and 241, and preferably 13, 30, 31, 37, 85, 129, 158 and 181, as compared to the amino acid sequence of SEQ ID NO: 10.

In some embodiments, the modified TPK polypeptide comprises the substitution, preferably conservative substitution, of 1, 2, 3, 4, 5, 6, 7, 8, 9, 10 or more amino acids at positions other than positions 35 and 241, and preferably 13, 30, 31, 37, 85, 129, 158 and 181, as compared to the amino acid sequence of SEQ ID NO: 11.

In some embodiments, the modified TPK polypeptide has catalytic activity of converting TM to TDP, which is at least 110%, 120%, 130%, 140%, 150%, 200%, 250%, 300% or more of that of the initial TPK polypeptide.

In some embodiments, the polynucleotide comprises a nucleotide sequence of SEQ ID NO: 15, 16 or 17, or a nucleotide sequence having at least 70%, 75%, 80%, 85%, 90%, 95%, 96%, 97%, 98% or 99% sequence identity to SEQ ID NO: 15, 16 or 17.

In some embodiments, the polynucleotide comprises a nucleotide sequence of SEQ ID NO: 15, or a nucleotide sequence having at least 70%, 75%, 80%, 85%, 90%, 95%, 96%, 97%, 98% or 99% sequence identity to SEQ ID NO: 15. In some embodiments, the polynucleotide comprises a nucleotide sequence of SEQ ID NO: 15, or a nucleotide sequence having at least 70%, 75%, 80%, 85%, 90%, 95%, 96%, 97%, 98% or 99% sequence identity to SEQ ID NO: 15; and the nucleotide sequence encodes an amino acid sequence comprising the substitution, preferably conservative substitution, of 1, 2, 3, 4, 5, 6, 7, 8, 9, 10 or more amino acids at positions other than positions other than positions 13, 30, 31, 37, 85, 129, 158 and 181, and preferably 241 as compared to SEQ ID NO: 9.

In some embodiments, the polynucleotide comprises a nucleotide sequence of SEQ ID NO: 16, or a nucleotide sequence having at least 70%, 75%, 80%, 85%, 90%, 95%, 96%, 97%, 98% or 99% sequence identity to SEQ ID NO: 16. In some embodiments, the polynucleotide comprises a nucleotide sequence of SEQ ID NO: 16, or a nucleotide sequence having at least 70%, 75%, 80%, 85%, 90%, 95%, 96%, 97%, 98% or 99% sequence identity to SEQ ID NO: 16; and the nucleotide sequence encodes an amino acid sequence comprising the substitution, preferably conservative substitution, of 1, 2, 3, 4, 5, 6, 7, 8, 9, 10 or more amino acids at positions other than positions 11 and 241, and preferably 13, 30, 31, 37, 85, 129, 158 and 181, as compared to SEQ ID NO: 10.

In some embodiments, the polynucleotide comprises a nucleotide sequence of SEQ ID NO: 17, or a nucleotide sequence having at least 70%, 75%, 80%, 85%, 90%, 95%, 96%, 97%, 98% or 99% sequence identity to SEQ ID NO: 17. In some embodiments, the polynucleotide comprises a nucleotide sequence of SEQ ID NO: 17, or a nucleotide sequence having at least 70%, 75%, 80%, 85%, 90%, 95%, 96%, 97%, 98% or 99% sequence identity to SEQ ID NO: 17; and the nucleotide sequence encodes an amino acid sequence comprising the substitution, preferably conservative substitution, of 1, 2, 3, 4, 5, 6, 7, 8, 9, 10 or more amino acids at positions other than positions 35 and 241, and preferably 13, 30, 31, 37, 85, 129, 158 and 181, as compared to SEQ ID NO: 11.

In some embodiments, the promoter is a neuron-specific promoter. In some embodiments, the promoter comprises a nucleotide sequence of SEQ ID NO: 3. In some embodiments, the promoter consists of a nucleotide sequence of SEQ ID NO: 3. In some embodiments, the promoter comprises a nucleotide sequence having at least 70%, 80%, 90%, 95%, 96%, 97%, 98%, 99% or more sequence identity to SEQ ID NO: 3. In some embodiments, the promoter consists of a nucleotide sequence having at least 70%, 80%, 90%, 95%, 96%, 97%, 98%, 99% or more sequence identity to SEQ ID NO: 3.

In some embodiments, the promoter is a eukaryotic strong promoter, for example, cytomegalovirus (CMV) promoter. In some embodiments, the promoter comprises a nucleotide sequence of SEQ ID NO: 7. In some embodiments, the promoter consists of a nucleotide sequence of SEQ ID NO: 7. In some embodiments, the promoter comprises a nucleotide sequence having at least 70%, 80%, 90%, 95%, 96%, 97%, 98%, 99% or more sequence identity to SEQ ID NO: 7. In some embodiments, the promoter consists of a nucleotide sequence having at least 70%, 80%, 90%, 95%, 96%, 97%, 98%, 99% or more sequence identity to SEQ ID NO: 7.

The expression cassette can comprise an additional control element, such as an enhancer, an intron, an mRNA stabilizing sequence and a polyadenylation signal sequence.

In some embodiments, the expression cassette further comprises an mRNA stabilizing sequence, for example a WPRE element. In some embodiments, the WPRE element comprises a nucleotide sequence of SEQ ID NO: 4. In some embodiments, the WPRE element consists of a nucleotide sequence of SEQ ID NO: 4. In some embodiments, the WPRE element comprises a nucleotide sequence having at least 70%, 80%, 90%, 95%, 96%, 97%, 98%, 99% or more identity to SEQ ID NO: 4. In some embodiments, the WPRE element consists of a nucleotide sequence having at least 70%, 80%, 90%, 95%, 96%, 97%, 98%, 99% or more identity to SEQ ID NO: 4.

In some embodiments, the WPRE element is downstream of the polynucleotide encoding TPK.

In some embodiments, the expression cassette further comprises a polyadenylation signal sequence, for example, but not limited to polyadenylation signal sequence of human growth factor gene and polyadenylation signal sequence of rabbit globulin gene. In some embodiments, the expression cassette further comprises polyadenylation signal sequence of human growth factor gene. In some embodiments, the polyadenylation signal sequence comprises a nucleotide sequence of SEQ ID NO: 5. In some embodiments, the polyadenylation signal sequence consists of a nucleotide sequence of SEQ ID NO: 5. In some embodiments, the polyadenylation signal sequence comprises a nucleotide sequence having at least 70%, 80%, 90%, 95%, 96%, 97%, 98%, 99% or more identity to SEQ ID NO: 5. In some embodiments, the polyadenylation signal sequence consists of a nucleotide sequence having at least 70%, 80%, 90%, 95%, 96%, 97%, 98%, 99% or more identity to SEQ ID NO: 5.

In some embodiments, the polyadenylation signal sequence is downstream of the WPRE element.

Non-limiting examples of the ITR in the genome of the rAAV comprise the ITR derived from AAV-1, AAV-2, AAV-3, AAV-4, AAV-5, AAV-6, AAV-7, AAV-8, AAV-9, poultry AAV, cattle AAV, canine AAV, horse AAV, primate AAV, non-primate AAV and sheep AAV In some embodiments, the ITR is ITR of AAV-2. In some embodiments, the 5' ITR is identical to the 3' ITR. In some embodiments, the 5' ITR is different from the 3' ITR.

In some embodiments, the 5' ITR and the 3' ITR are both ITR130, for example, comprising a nucleotide sequence of SEQ ID NO: 6. In some embodiments, the 5' ITR and the 3' ITR consists of a nucleotide sequence of SEQ ID NO: 6, respectively. In some embodiments, the 5' ITR and the 3' ITR comprise a nucleotide sequence having at least 70%, 80%, 90%, 95%, 96%, 97%, 98%, 99% or more identity to SEQ ID NO: 6, respectively. In some embodiments, the 5' ITR and the 3' ITR consist of a nucleotide sequence having at least 70%, 80%, 90%, 95%, 96%, 97%, 98%, 99% or more identity to SEQ ID NO: 6, respectively.

In some embodiments, the genome of the rAAV comprises, from 5' to 3', SEQ ID NO: 3, SEQ ID NO: 2, SEQ ID NO: 4, SEQ ID NO: 5. In some embodiments, the genome of the rAAV comprises, from 5' to 3', SEQ ID NO: 6, SEQ ID NO: 3, SEQ ID NO: 2, SEQ ID NO: 4, SEQ ID NO: 5, SEQ ID NO: 6.

In some embodiments, the genome of the rAAV comprises, from 5' to 3', SEQ ID NO: 3, SEQ ID NO: 14, SEQ ID NO: 4, SEQ ID NO: 5. In some embodiments, the genome of the rAAV comprises, from 5' to 3', SEQ ID NO: 6, SEQ ID NO: 3, SEQ ID NO: 14, SEQ ID NO: 4, SEQ ID NO: 5, SEQ ID NO: 6.

In some embodiments, the genome of the rAAV comprises, from 5' to 3', SEQ ID NO: 3, SEQ ID NO: 15, SEQ ID NO: 4, SEQ ID NO: 5. In some embodiments, the genome of the rAAV comprises, from 5' to 3', SEQ ID NO: 6, SEQ ID NO: 3, SEQ ID NO: 15, SEQ ID NO: 4, SEQ ID NO: 5, SEQ ID NO: 6.

In some embodiments, the genome of the rAAV comprises, from 5' to 3', SEQ ID NO: 3, SEQ ID NO: 16, SEQ ID NO: 4, SEQ ID NO: 5. In some embodiments, the genome of the rAAV comprises, from 5' to 3', SEQ ID NO: 6, SEQ ID NO: 3, SEQ ID NO: 16, SEQ ID NO: 4, SEQ ID NO: 5, SEQ ID NO: 6.

In some embodiments, the genome of the rAAV comprises, from 5' to 3', SEQ ID NO: 3, SEQ ID NO: 17, SEQ ID NO: 4, SEQ ID NO: 5. In some embodiments, the genome of the rAAV comprises, from 5' to 3', SEQ ID NO: 6, SEQ ID NO: 3, SEQ ID NO: 17, SEQ ID NO: 4, SEQ ID NO: 5, SEQ ID NO: 6.

In some embodiments, the genome of the recombinant lentivirus comprises, from 5' to 3', SEQ ID NO: 7, SEQ ID NO: 2, SEQ ID NO: 4, SEQ ID NO: 5.

In some embodiments, the genome of the recombinant lentivirus comprises, from 5' to 3', SEQ ID NO: 7, SEQ ID NO: 14, SEQ ID NO: 4, SEQ ID NO: 5.

In some embodiments, the genome of the recombinant lentivirus comprises, from 5' to 3', SEQ ID NO: 7, SEQ ID NO: 15, SEQ ID NO: 4, SEQ ID NO: 5.

In some embodiments, the genome of the recombinant lentivirus comprises, from 5' to 3', SEQ ID NO: 7, SEQ ID NO: 16, SEQ ID NO: 4, SEQ ID NO: 5.

In some embodiments, the genome of the recombinant lentivirus comprises, from 5' to 3', SEQ ID NO: 7, SEQ ID NO: 17, SEQ ID NO: 4, SEQ ID NO: 5.

The rAAV can be AAV-1, AAV-2, AAV-3, AAV-4, AAV-5, AAV-6, AAV-7, AAV-8, AAV-9, poultry AAV, cattle AAV, canine AAV, horse AAV, primate AAV, non-primate AAV and sheep AAV In some embodiments, the rAAV is an rAAV of serotype AAV_PHP eB.

It is known in the field that rAAV is packaged using a system containing three plasmids, comprising: 1) a transgenic plasmid containing the genome of rAAV encoding the gene product of interest; ii) a packaging plasmid encoding REP and/or CAP proteins; and iii) an helper plasmid (see, for example, Crosson SM et al., Helper-free Production of Laboratory Grade AAV and Purification by Iodixanol Density Gradient Centrifugation. Mol Ther Methods Clin Dev. 2018; 10:1-7). The packaging involves the introduction of the system into host cells.

The lentivirus packaging system is known in the field, which consists of a transfer vector (transgenic plasmid), an envelope vector encoding selected envelope proteins, and a packaging vector which provides lentivirus proteins (such as lentivirus GAG and POL proteins, especially mutated POL proteins to prevent integration) according to well-known methods in the field. The package of lentivirus is involved in introducing the system into host cells.

Provided is also a host cell, comprising the polynucleotide, vector, rAAV or recombinant lentivirus according to the present disclosure. The host cell is referred to as a "packing cell" when being used for packing the rAAV or recombinant lentivirus virion. In some embodiments, the host cell is stably genetically modified with the polynucleotide or vector according to the present disclosure. In other embodiments, the host cell is transiently genetically modified with the polynucleotide or vector according to the present disclosure.

Certain technologies, including but not limited to electroporation, calcium phosphate precipitation, liposome mediated transfection or the like are used to stably or transiently introduce the polynucleotide or vector according to the present disclosure into host cells.

For stable transformation, the polynucleotide or vector according to the present disclosure will typically further comprise a selectable marker, such as any of several well-known selectable markers, e.g., neomycin resistance or the like.

The host cell according to the present disclosure may be derived from a mammalian cell. Suitable mammalian cell comprises but not limited to a primary cell and cell line, wherein suitable cell line comprises but not limited to 293 cell, COS cell, HeLa cell, Vero cell, 3T3 mouse fibroblast, C3H10T1/2 fibroblast, CHO cell or the like. Non-limiting examples of the host cell comprise (for example) HeLa cell (e.g., ATCC No. CCL-2), CHO cell (e.g., ATCC No. CRL9618, CCL61, CRL9096), 293 cell (e.g., ATCC No. CRL-1573), Vero cell, N1H3T3 cell (e.g., ATCC No. CRL-1658), Huh-7 cell, BHK cell (e.g., ATCC No. CCL10), PC12 cell (ATCC No. CRL1721), COS cell, COS-7 cell (ATCC No. CRL1651), RAT1 cell, mouse L cell (ATCC No. CCL1.3), human embryonic kidney (HEK) cell (ATCC No. CRL1573), HLHepG2 cell or the like.

### V Pharmaceutical composition

Provided is a pharmaceutical composition, comprising the polynucleotide, vector, rAAV or recombinant lentivirus according to the present disclosure, and a pharmaceutically acceptable carrier, diluent, excipient or buffer. In some embodiments, pharmaceutically acceptable carrier, diluent, excipient or buffer is suitable for use in human.

Such excipient, carrier, diluent and buffer comprise any agent which can be applied without abnormal toxicity. The pharmaceutically acceptable excipient comprises but not limited to liquid such as water, saline, glycerol and ethanol, wherein the pharmaceutically acceptable salts, for example mineral acid salts, e.g., hydrochloride, hydrobromate, phosphate, sulfate or the like; and organic acid salts, e.g., acetate, propionate, malonate, benzoate or the like, may be contained. In addition, auxiliary substances like wetting agent or emulsifier, pH buffering substance or the like may be present in such carriers. Various pharmaceutically acceptable excipients are known in the field without discussion in detail herein. The pharmaceutically acceptable excipients have been described in detail in various publications, comprising (for example) A. Gennaro (2000) "Remington: The Science and Practice of Pharmacy," 20th Edition, Lippincott, Williams, & Wilkins; Pharmaceutical Dosage Forms and Drug Delivery Systems (1999) Editing by HC Ansel et al, 7th edition, Lippincott, Williams, & Wilkins; and Handbook of Pharmaceutical Excipients (2000) AH Kibbe et al., 3rd edition, Amer. Pharmaceutical Assoc.

### VI. Prevention and treatment of diseases

Provided is a method for promoting glycometabolism, or preventing or treating a glucose metabolism disorder, preferably a glucose metabolism disorder in brain, comprising administering a subject in need thereof the polynucleotide, vector, rAAV, recombinant lentivirus or pharmaceutical composition according to the present disclosure. Provided is a method for treating or preventing Alzheimer's disease, comprising administering a subject or patient in need thereof the polynucleotide, vector, rAAV or recombinant lentivirus, or pharmaceutical composition according to the present disclosure. In some embodiments, the polynucleotide, vector, rAAV, recombinant lentivirus or pharmaceutical composition is administered intravenously, intracerebrally, or intrathecally. In some embodiments, the subject or patient is a human.

Provided is also use of the polynucleotide, vector, rAAV or recombinant lentivirus, or pharmaceutical composition according to the present disclosure in the manufacture of a medicament for promoting glycometabolism, or preventing or treating a glucose metabolism disorder, preferably a glucose metabolism disorder in brain. Provided is also use of the polynucleotide, vector, rAAV, recombinant lentivirus or pharmaceutical composition according to the present disclosure for the manufacture of a medicament for treating or preventing Alzheimer's disease. In some embodiments, the medicament is administered intravenously, intracerebrally, or intrathecally. In some embodiments, the medicament is administered to human.

Provided is further the polynucleotide, vector, rAAV, recombinant lentivirus or pharmaceutical composition according to the present disclosure, for use in promoting glycometabolism, or preventing or treating a glucose metabolism disorder, preferably a glucose metabolism disorder in brain. Provided is also the polynucleotide, vector, rAAV, recombinant lentivirus or pharmaceutical composition, for use in treating or preventing Alzheimer's disease. In some embodiments, the polynucleotide, vector, rAAV, recombinant lentivirus or pharmaceutical composition is administered intravenously, intracerebrally, or intrathecally. In some embodiments, the polynucleotide, vector, rAAV, recombinant lentivirus or pharmaceutical composition is administered to human.

The prevention or treatment of Alzheimer's disease comprises preventing, reducing, or eliminating symptoms of AD, such as AD like neurodegenerative lesions, brain atrophies, pathological changes of Aβ and tau, neuroinflammation and neurovascular disorders, or delaying the progression of AD.

AD involves damages to a number of brain regions, and the virus according to the present disclosure can selectively and efficiently penetrate the blood-brain barrier, widely affecting multiple brain regions. In clinical application, the rAAV according to the present disclosure can be administered via direct injection across the blood-brain barrier (such as intracerebral injection or intrathecal injection), or via peripheral venous injection, which can also achieve the same effect as direct injection into the brain. In addition, the difference between the present disclosure and other gene therapies is that it only mediates the compensatory expression of TPK through exogenously, does not involve endogenous TPK, much less the editing of other genes, which can greatly reduce other risks. More importantly, the present disclosure introduces highly active exogenous TPK, achieving beneficial therapeutic effects.

GSK3β is a serine/threonine kinase which is highly conserved in evolution, and can act on other pathways in addition to regulating glycogen synthase activity, to regulate cell differentiation, proliferation, survival, and apoptosis. It has been reported that GSK3β activation can lead to AD onset by promoting the phosphorylation of tau protein, increasing Aβ deposition, activating microglia, damaging neuronal generation, inhibiting LTP or the like (Elisabetta Lauretti, Ozlem Dincer, Domenico Praticò, Glycogen synthase kinase-3 signaling in Alzheimer's disease, Biochimica et Biophysica Acta (BBA) - Molecular Cell Research,Volume 1867, Issue 5, 2020). Advantageously, the rAAV according to the present disclosure expresses TPK *in vivo* (such as in brain), which mediates the "TPK-TDP glycometabolism" pathway and GSK3β Pathway interaction, enhances phosphorylated GSK3β (p-GSK3β) level and p-GSK3β/GSK3β ratio, inhibits GSK3β activity, and provides beneficial therapeutic effects for AD.

### Examples

Through the following examples, those skilled in the art will understand the present disclosure more clearly. It should be understood that the following Examples are intended to illustrate exemplary embodiments of the present disclosure, rather than limit the scope thereof. Unless otherwise specified, the methods used herein are conventional in the art, and the experimental materials as used are commercially available.

### Example 1 Preparation of rAAV and recombinant lentivirus

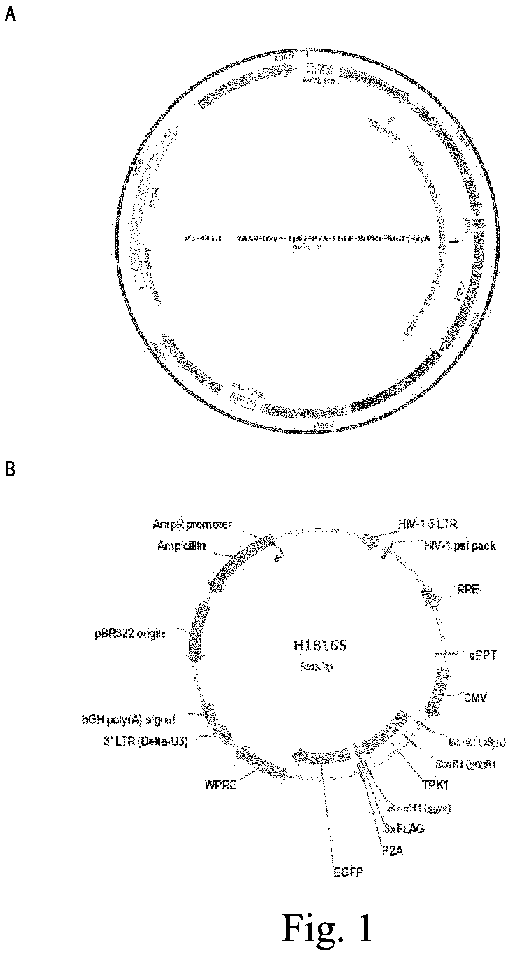

A CRO company Wuhan BrainVTA was entrusted to prepare laboratory level rAAV encoding TPK (serotype AAV_PHP eB), referred to as AAV-TPK, of which the genome comprises: i) a neuron-specific promoter (SEQ ID NO: 3), ii) a nucleotide sequence encoding mouse TPK (SEQ ID NO: 2) and a WPRE element (SEQ ID NO: 4). The plasmid containing the genome of the rAAV was shown in Fig. 1A.

A CRO company Shanghai OBiO was entrusted to prepare laboratory level lentivirus encoding TPK, referred to as TPK lentivirus, of which the genome comprises: i) eukaryotic expression promoter (SEQ ID NO: 7), ii) nucleotide sequence encoding mouse TPK (SEQ ID NO: 2) and WPRE element (SEQ ID NO: 4). The plasmid containing the genome of the lentivirus was shown in Fig. 1B.

### Example 2 Overexpression of TPK and increased TPD content in neuronal cells infected with TPK lentivirus

The purpose of this example was to preliminarily evaluate the efficacy of gene therapy through cellular level experiments.

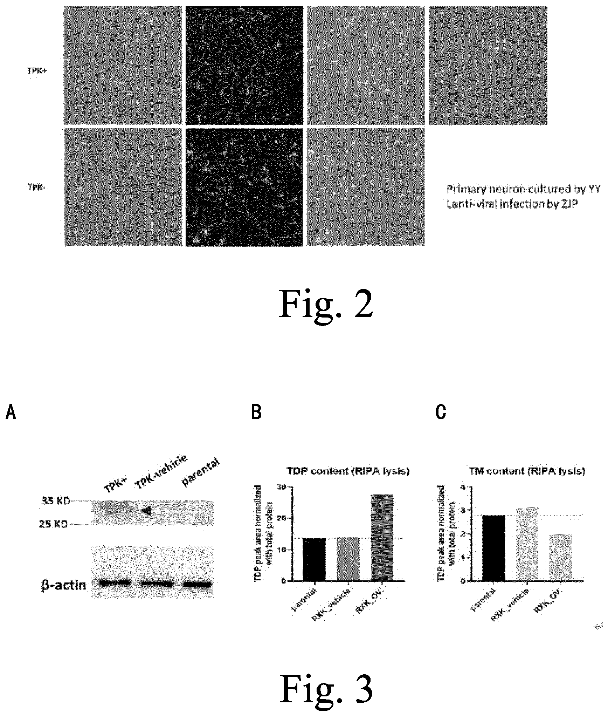

The cerebral cortex of neonatal mice (purchased from Zhejiang Charlesriver) was digested with trypsin (Thermofisher/Gibco, Catalog No: 25200072) to obtain primary neurons, which were cultured in 6-well plates (1.5 × 10⁶/well) for 4 days (10%FBS + 10%F12 + 1%Glutemax + 78% DMEM + 1% 100X penicillin-streptomycin double antibody (5000 U/mL) on Day 1, followed by Neurobasal medium+2%B27+1%Glutemax+1% 100X penicillin-streptomycin double antibody (5000 U/mL), wherein the above reagents were formulated in volume ratio and all purchased from Thermofisher/Gibco). The culture medium was removed, and the lentivirus suspension prepared in Example 1 (1.5mL in PBS with MOI of 5) was added for infection for 8 h, wherein the lentivirus encoding GFP without TPK encoding sequence was used as control. The liquid was removed, and fresh culture medium was added for 7 days of cultivation. GFP expression was observed with fluorescence microscopy. As shown in Fig. 2, a large number of infected cells expressed GFP, indicating high infection rate of the virus.

The culture medium was removed, and the cells were washed three times with pre-cooled PBS, 5 min per time. 100 µL of cell lysate was added to each well, containing RIPA (purchased from Beyotime), phosphatase inhibitor (purchased from Bimake), and protease inhibitor (purchased from Sigma) in a ratio of 100:1:1 (v: v: v). All cells were scraped off and the cell suspension was transferred to 1.5mL centrifuge tube on ice. 50 µL of cell lysate was added to each well to flush the bottom of the well and transferred to the same centrifuge tube. 2 grinding steel balls were added to each tube for grinding (60Hz, 60s, -10°C); centrifuge was conducted at 13000 rpm, 4 °C for 15 min, and the supernatants were kept on ice.

The total protein concentration in the supernatant was determined by BCA method. After the extracted total protein was denatured at 95°C, TPK levels in the samples were analyzed by Western Blot. Briefly, after denaturation, the sample was loaded into the wells of polyacrylamide gel (15 µg protein per well for the sample to be tested, 5 µL for protein size indicator (Marker)), and the membrane was transferred after electrophoresis was completed, and then, the membrane corresponding to the area of target protein size was cut according to Marker (TPK was 25-35KD, β-Actin was 40-55KD). The membrane was blocked at room temperature for 1 h with blocking solution (purchased from Takara, Catalog No: T7131A), and then, incubated with the following primary antibodies at 4°C over night: anti-TPK1 antibody (purchased from Proteintech, Catalog No: 10942-1-AP) and mouse anti-β-Actin antibody (purchased from Beyotime, Catalog No: AF0003). The primary antibodies were recovered, and the membranes were washed three times with 1X TBST, 5 mins each time. Corresponding secondary antibodies horseradish peroxidase labeled goat anti-rabbit IgG (H+L) (purchased from Beyotime, Catalog No: A0208); horseradish peroxidase labeled goat anti-mouse IgG(H+L) (purchased from Beyotime, Catalog No: A0216) were added, followed by the incubation at room temperature for 2 h, washing for three times with 1xTBST, and development with horseradish peroxidase kit (purchased from Thermo Fisher, Catalog No: 34095). As shown in Fig. 3A, the cells infected with TPK lentivirus significantly overexpressed TPK.

100 µL of supernatants were taken, to which equal volume of pre-cooled 5.4% perchloric acid (PCA) was add, followed by blending by shaking the well, and the centrifugation at 13,000 rpm, 4 °C for 15 min. HPLC analysis of the supernatants was conducted using the equipments and conditions shown in Table 1.

**Table 1**

| Instrument and software | Manufacture | Type | |
|---|---|---|---|
| Liquid chromatography system | Thermo Fisher | Ultimate3000 | |
| (QuatPump/Column oven/Auto Sampler/Vacuum degasser/Fluorescence detector) | Thermo Fisher | Ultimate3000 | |
| Data acquisition software | Thermo Fisher | ES Chromeleon 7.2.10 | |
| Chromatographic column | Agilent Technologies | C₁₈s(4.6×150 mm) | |
| Detector | Fluorescence detector (FLD) | | |
| HPLC conditions | | | |
| Detecting conditions | Ex=367 nm, Em=435 nm | | |
| Mobile phase and Flow rate | 20 mM Ammonium | | |
| | Time min | acetate aqueous solution | Methanol |
| | 0-1.3 | 90 | 10 |
| | 4.2-6.2 | 61 | 39 |
| | 8.6-9.6 | 12 | 88 |
| | 9.7-10 | 90 | 10 |
| | Flow rate: 1 mL/min | | |

As shown in Fig. 3B, TDP content in cells infected with TPK lentivirus was significantly higher than those in uninfected cells and cells infected control virus, while TM content in AAV-TPK cells was significantly lower than those in uninfected cells and cells infected control virus (Fig. 3C). The results showed that overexpression of TPK significantly promoted the conversion of TM to TDP in cells infected with TPK lentivirus, with conversion rate about 2 times greater than that of the control group.

### Example 3: Overexpression TPK and elevated TM/TDP conversion rate in brain of mice injected with AAV-TPK via tail vein

The purpose of this example is to verify whether gene therapy can significantly improve the expression of TPK protein in the brain of C57BL/6J mice and increase TM/TDP conversion rate in brain of mice, thereby enhancing glycometabolism by tail vein injection of AAV to mice.

After 3 days of adaptive feeding, adult C57BL/6J mice (2 months) (purchased from Zhejiang Charlesriver) were randomly divided into two groups (TPK group and control group, n=3), to each of which the suspension of AAV-TPK (TPK group) and control rAAV (the same as TPK-AAV except for the absence of TPK coding sequence) with titer of 5.0E+12vg/mL (formulated with physiological saline) was administered by tail vein injection, 200 µL per mouse. Specifically, the mice were fixed on a tail vein injection fixator, with the tail wiped with alcohol cotton ball for disinfection. 1 ml syringe was used to inject 200 µL of virus suspension into mouse tail vein, and the injection site was pressed with dry cotton ball for 30 s after injection to prevent virus leakage. The mice were raised in the experimental area for 4 weeks, and the weight and food intake were recorded weekly.

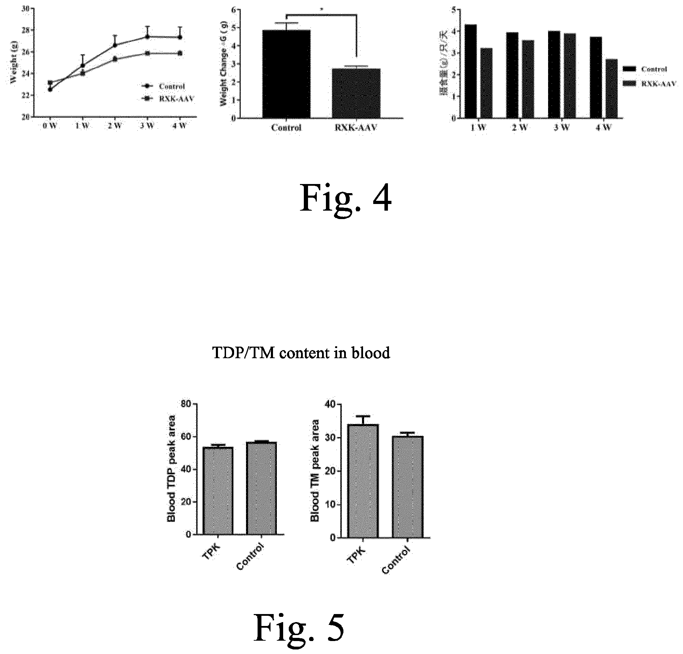

As shown in Fig. 4, after AAV-TPK injection via tail vein in adult mice, the weight gains were significantly smaller than those of the control group, and the food intake showed a decreasing trend compared to the control group.

After 4 weeks of feeding, 3 mL EDTA anticoagulant tubes were used to collect whole blood of the mice, which were immediately placed on ice. The following procedures were conducted on ice: in 10 min, 0.3 mL of whole blood sample was transferred to 2mL centrifuge tube, to which were added 30 µ L of pure water, and then 0.3 mL of pre-cooled PCA (5.7%) to precipitate protein. After about 30 min of ice bath, the samples were centrifuged at 4°C, 12000 rpm for 10 min. The supernatants were taken and analyzed for TDP and TM contents by HPLC as described in Example 2

As shown in Fig. 5, after AAV-TPK injection via tail vein in adult mice for 4 weeks, there was no significant change in TDP/TM contents in the peripheral blood.

The mice were euthanized and then subjected to cardiac perfusion with pre-cooled PBS. The mice brain tissues were collected (cerebellum, brainstem, and olfactory bulb parts were removed, and left and right brains were separated).

The right brain was fixed with 4% paraformaldehyde (PFA) and immunofluorescence staining was performed. Specifically, after being fixed with 4% PFA for 12-24 h, the right brain was dehydrated and sugar-precipitated with 30% sucrose for 2 days. After sugar precipitation, the right brain was embedded in frozen section embedding agent (OCT) and frozen at -80°C. Cryoslicer was used to slice the embedded right brain into thin sections of 30 µm, with the slice angle being longitudinal (coronal) from the olfactory bulb to the cerebellum. The slices were washed 3-5 times with 1X PBS to remove OCT. 0.5% Triton was added at room temperature for membrane breaking for 30 min, then replaced with 3% BSA (bovine serum albumin) and blocked at room temperature for 1 h. Primary antibody (anti-GFP antibody, purchased from Avessellabs, Catalog No. GFP-1020) was added and incubated was conducted overnight at 4 °C. The primary antibody was recovered and cleaned 3 times with 1X PBS, 5 min per time. Secondary antibody Goat anti-Rabbit IgG (H+L) Cross-Adsorbed Secondary Antibody, Alexa Fluor 488, (purchased from IInvitrogen, Catalog No. A11008) was added and incubation was conducted in dark for 2 h. Finally, the thin sections were applied on the glass slide and dried slightly, and then, the mounting medium was added dropwise for mounting. Nikon Ti2E inverted microscope was used for imaging and photography.

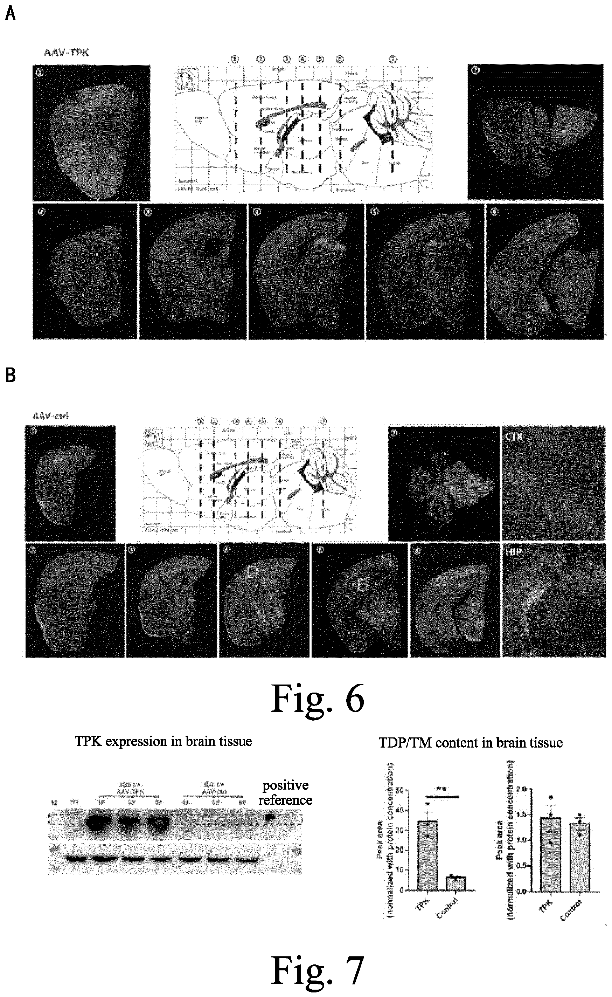

As shown in Fig. 6, 4 weeks after tail vein injection of AAV-TPK in adult mice, the polypeptide encoded by rAAV virus could be widely expressed in brain tissues.

The left brain or liver tissues (left upper lobe) were placed in 1.5ml of cell lysis solution (see Example 2), and lysed using freeze grinder (70Hz, 60s, twice, with interval of 20 s); then centrifuged at 13000 rpm, 4 °C for 15 min, and the supernatants were taken. Western Blot and HPLC analysis were conducted as described in Example 2 to detect TPK expression and TDP and TM contents in the brain.

As shown in Fig. 7, 4 weeks after tail vein injection of AAV-TPK in adult mice, TPK expression in brain tissues was significantly increased, TDP content was significantly increased, and TM content was comparable to that of the control.

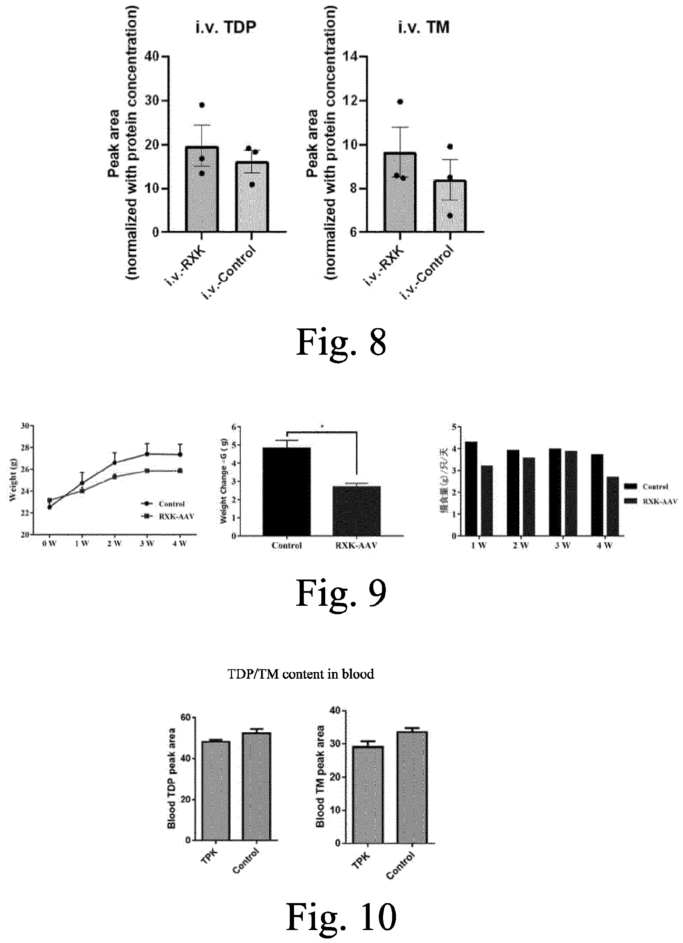

As shown in Fig. 8, the TM and TDP in liver were not significantly changed, indicating that the virus well good brain specificity, specifically targeting brain neurons without affecting peripheral tissues. The side effects of the virus were completely controllable.

### Example 4: TPK overexpression and elevated TM/TDP conversion rate in brain of mice injected with AAV-TPK in lateral ventricle

The purpose of this example is to verify whether injection of AAV-TPK virus in lateral ventricle of mice can significantly increase expression of TPK protein in the brain of C57BL/6J mice and increase TM/TDP conversion rate in mouse brain, thereby enhancing glycometabolism.

After 3 days of adaptive feeding, adult C57BL/6J mice (2 months) (purchased from Zhejiang Charlesriver) were randomly divided into two groups (TPK group and control group, n=5), for stereotactic injection of AAV in lateral ventricle of the brain.

The concentration of virus stock solution as used was 1.0E+13vg/1mL, and the injection volume in lateral ventricle of mice was 4.0E+10vg/4 µL (2 µL for each of the left and right lateral ventricles) per mouse.

After anesthetizing adult mice with Zoletil 50, the hair between ears and eyes was shaved off and the mice were fixed on brain stereotaxic device. The skin at the brain region of the mice was wiped with an alcohol cotton ball and disinfected. Scissors were used to open the skin and expose the skull. Surface of the skull was wiped to dryness with a dry cotton swab until the anterior fontanelle point was clearly visible. The coordinates of the anterior fontanelle point were designated as 0 (AP: -0.2mm, L/R: ±1.0mm, H: 2.5mm), and the drilling point was marked. A 0.5mm drill bit was used to drill a hole in the skull, and 2 µL of AAV virus was inhaled with a 10 µL micro injection needle. The depth of the needle was 2.5mm, with the skull surface as the Z-axis 0 point. Injection speed: 0.2 µL/min, and the injection was ceased for 5 min, and the injection needle was slowly removed. After the skin was sutured, the mice were placed in a cage containing heating pad and fed in the experimental area of feeding cage for 4 weeks after awakening. The weight and food intake were recorded weekly.

As shown in Fig. 9, there was no significant difference in the body weight and food intake between adult mice injected with AAV-TPK in lateral ventricle and the control group.

After 4 weeks of feeding, the samples were collected and TDP and TM levels in the blood, the expression of polypeptide encoded by rAAV virus in brain, and TPK expression level and TDP and TM levels in brain and liver tissues were detected as described in Example 3.

As shown in Fig. 10, there was no significant change in peripheral blood TDP/TM contents 4 weeks after AAV-TPK injection in the lateral ventricle of adult mice.

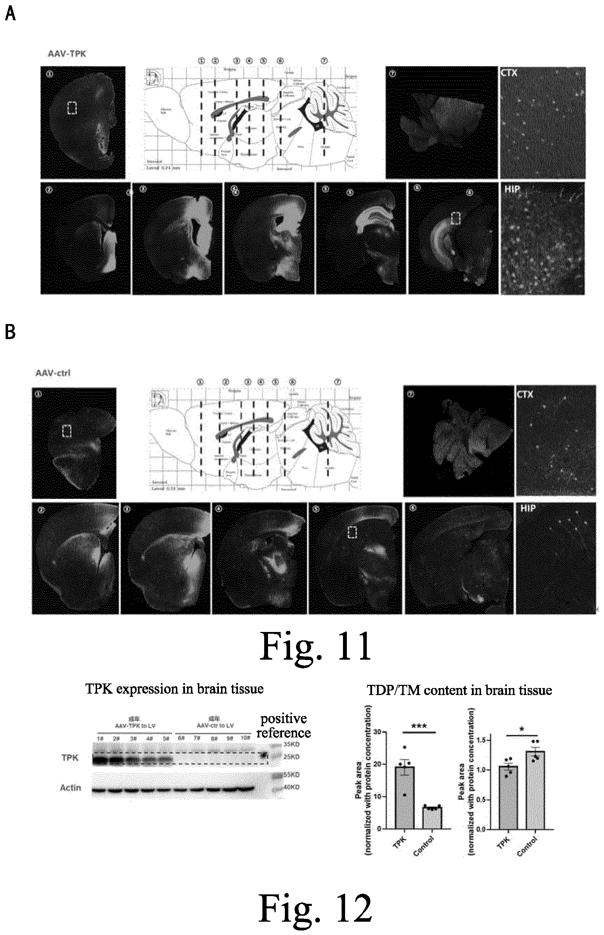

As shown in Fig. 11, the gene products encoded by rAAV virus were strongly expressed around the lateral ventricle and also expressed in some other brain regions 4 weeks after rAAV injection in the lateral ventricle of adult mic,.

As shown in Fig. 12, the TPK expression in brain tissues was significantly increased, TDP content was significantly increased, and TM content was decreased, indicating increase in TM/TDP conversion rate4 weeks after AAV-TPK injection in the lateral ventricle of adult mice, as compared with the control group.

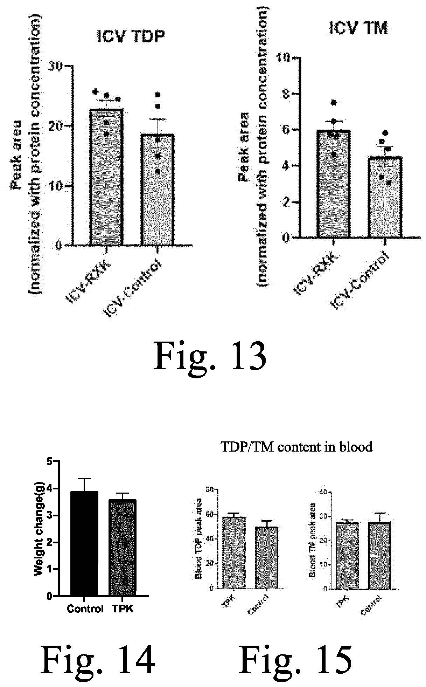

As shown in Fig. 13, the TM and TDP in liver were not significantly changed, indicating that the virus had well brain specificity, specifically targeting brain neurons without affecting peripheral tissues. The side effects of the virus were completely controllable.

### Example 5: TPK overexpression and elevated TM/TDP conversion rate in brain of mice developed from embryos injected with AAV-TPK into the lateral ventricle

The purpose of this example is to verify whether gene therapy by direct delivery of AAV in lateral ventricle of fetal mice can significantly increase the TPK expression in C57BL6/J mice brain neurons, improve the TM/TDP conversion rate in neurons, thereby enhancing glycometabolism.

C57BL6/J mice (embryo 13.5 days, E13.5) purchased from Zhejiang Charlesriver were adaptively raised for 1 day (E14.5) and randomly divided into two groups (n=3): TPK group (injected with AAV-TPK) and control group (injected with control virus).

The mice were deeply anesthetized and then placed with abdomen flat, and abdomen of the pregnant mice was wiped with a tissue dipped in 75% alcohol. The hair in the center of abdomen were cut off, and then abdomen of the pregnant mice was wipe with a tissue dipped in PBS to remove residual alcohol and broken hair.

The surgical instruments were wiped with 75% alcohol for disinfection. The skin was cut along middle of the pregnant mouse abdomen with scissors as about 1.5 cm, and the pregnant mouse peritoneum was cut along white line of the abdomen, exposing the abdominal cavity. The embryo was carefully removed from abdominal cavity of the pregnant mouse using tweezers and laid on the abdominal cavity of the pregnant mouse (during the following surgical procedures, the abdominal cavity and embryo surface were kept moist with PBS). The glass electrode which has absorbed rAAV virus suspension was gently punctured into embryonic brain, and the virus suspension was transferred to lateral ventricle of the embryo by blowing air, wherein 0.5~1 µL (5.0E+9~1.0E+10 vg) of virus suspension was injected into each lateral ventricle of each embryo. The embryo was placed back into abdominal cavity of the pregnant mouse and the mouse peritoneum and skin were sutured in sequence with suture lines. After suture, lincomycin lidocaine gel was applied for analgesia to prevent wound inflammation and infection, and then the mouse was placed on the heating pad until wake up and transferred back to the cage to continue feeding.

After birth, the offspring mouse was irradiated with excitation light of 480 nm on head to test the results of virus injection, and the mouse with successful virus injection was retained. After 21 days of feeding, three mice were treated as described in Example 3 to collect samples and detect levels of TDP and TM in blood, expression of polypeptide encoded by rAAV virus in the brain, expression level of TPK and TDP and TM levels in brain and liver tissues. Other 12 mice were weaned, and the weight were recorded. They were divided into cages for two weeks and the weight was recorded again.

As shown in Fig. 14, there was no significant difference in weight change within 2 weeks after weaning between mice developed from embryos injected with AAV-TPK and the control group.

As shown in Fig. 15, there was no significant change in peripheral blood TDP/TM contents of mice developed from embryos injected with AAV-TPK.

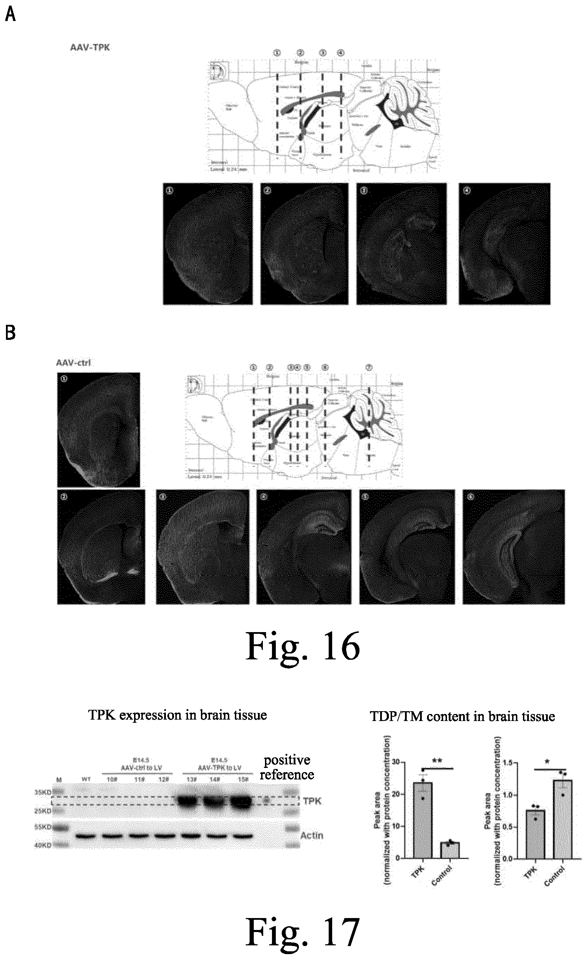

As shown in Fig. 16, in mouse developed from embryos injected with rAAV, the gene products encoded by rAAV virus were widely expressed in cortex and hippocampus, as well as in some other brain regions.

As shown in Fig. 17, compared with the control, TPK expression and TDP content in brain tissues of mouse developed from embryos injected with AAV-TPK were significantly increased, while TM content was decreased, indicating increase in TM/TDP conversion rate.

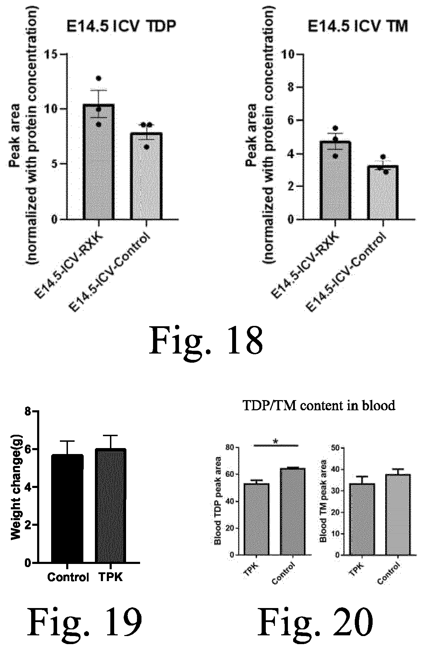

As shown in Fig. 18, the TM and TDP in liver were not significantly changed, indicating that the virus had good brain specificity, specifically targeting brain neurons without affecting peripheral tissues. The side effects of the virus were completely controllable.

### Example 6: TPK overexpression and elevated TM/TDP conversion rate in brain of neonatal mouse with stereotactic injection of AAV-TPK

The purpose of this example is to verify whether direct delivery of AAV-TPK to lateral ventricle (Intracerebral ventricular, ICV) and (Intraparenchymal, IP) of neonatal mouse (Postnatal 0 day, P0 mouse) can significantly increase TPK expression in C57BL6/J mouse brain neurons, improve TM/TDP conversion rate in neurons, thereby enhancing glycometabolism.

C57BL6/J mice (embryo 18.5 days, E18.5) were purchased from Shanghai JieSiJie Laboratory Animal Co., Ltd., in total of 11. The experiments were conducted after the birth of neonatal mice.

Lateral ventricular injection site: 1 mm forward, 1 mm left and right, and 1 mm deep from Lambda. 800 nL of virus suspension (1.0E+13vg/mL) was injected in lateral ventricles on both sides.

Hippocampal injection site ①: 0.7 mm forward, 1.2 mm left and right, and 0.9 mm deep from Lambda. Hippocampus injection site ②: 0.1 mm forward, 2.2 mm left and right, and 1.4 mm deep from Lambda. Injections were conducted at two sites into both sides of the hippocampus, in total of four, with each injection of 200 nL virus suspension (1.0E+13vg/mL).

After the glass electrode was filled with paraffin oil, it was fixed on microinjector with a hot melt gun. Needle of the microinjector was inserted into about one-third in the glass electrode, and the microinjector was fixed to automatic injection pump.

Body of the neonatal mouse was buried in ice without covering its nose to allow breath for about 3 min. After the body changed from pink purple to slightly white without struggle, the P0 mouse was removed from the ice and placed on ice surface for subsequent experiments.

Virus suspension was sucked into glass electrode through injection pump. The neonatal mice deeply anesthetized by ice treatment was fixed on horizontal vessel with medical tapes, keeping the head horizontal.

Under stereomicroscope, the posterior fontanelle (Lambda) of mouse head was designated as the origin, the syringe was moved to the designated position through operating arm, and the up and down positions of the syringe were adjusted through vertical operating arm (Z-axis). The position where the needle slightly sank after touching the head was set as point 0, and the needle was sent downwards to the designated position. Finally, the virus was automatically delivered to brain of neonatal mouse through an injection pump controller. After the injection was completed, the needle was allowed to stay in brain of the neonatal mouse for about 3 min, and then slowly removed.

After injection, the neonatal mouse was quickly transferred to heating pad. After body temperature of the neonatal mouse recovered and it began to move, the animal was placed back into mother mouse cage and covered with cage padding for a period of time to be stained with smell. The stereotactic experiment ended when the mother mouse retrieved the neonatal mouse into the nest initiatively.

After 21 days of feeding, 3 mice of each treatment were treated as described in Example 3 to collect samples and detect TDP and TM levels in blood, expression of polypeptide encoded by rAAV virus in brain, TPK expression levels and TDP and TM levels in brain and liver tissues. Other mice (n=11 injection in lateral ventricle and n=8 injection in hippocampus) were weaned and the weight was recorded. The animals were then divided into cages and fed for two weeks and the weight was recorded again.

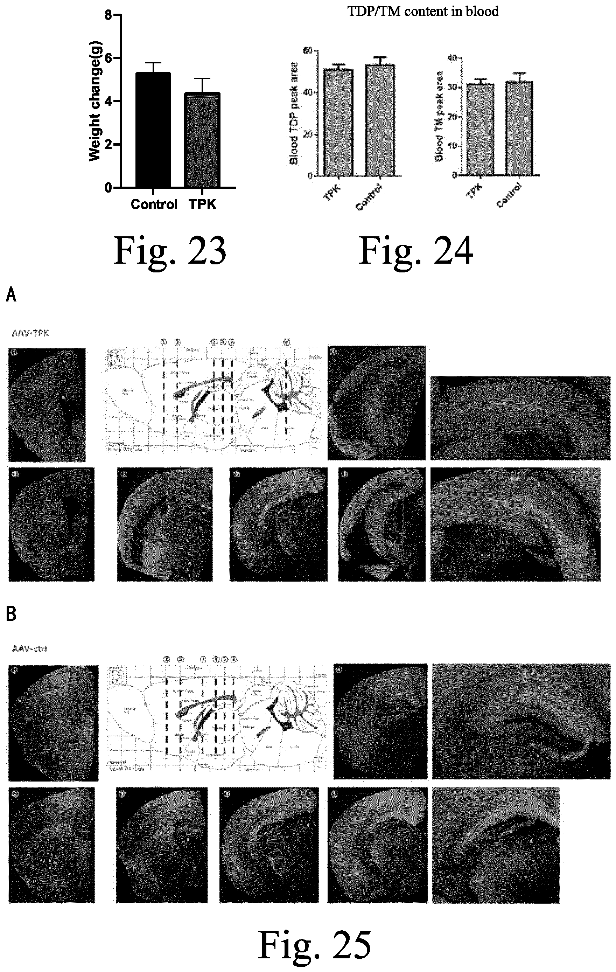

As shown in Fig. 19, 3 weeks after lateral ventricular injection of AAV-TPK, there was no significant change in body weight within 2 weeks after weaning compared to the control group in P0 mice, and 3 weeks after hippocampal injection of AAV-TPK, there was no significant change in body weight within 2 weeks after weaning compared to the control group in P0 mice (Fig. 23).

As shown in Fig. 24, 3 weeks after AAV-TPK injection in hippocampus in P0 mice, there were no significant changes in peripheral blood TDP and TM contents.

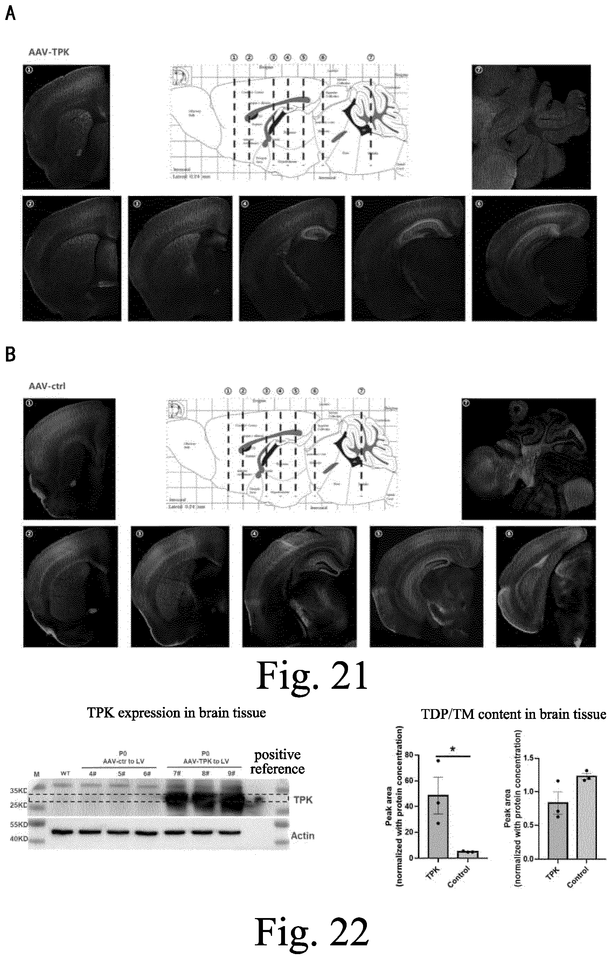

As shown in Fig. 21, 3 weeks after rAAV injection in lateral ventricle in P0 mice, the gene products encoded by rAAV were widely expressed in cortex and hippocampus, as well as in some other brain regions, and 3 weeks after injection of rAAV in hippocampus in P0 mice, the gene products encoded by rAAV were strongly expressed in the hippocampus as well as in some other brain regions (Fig. 25).

As shown in Fig. 22, 3 weeks after lateral ventricular injection of rAAV in P0 mice, TPK expression and TDP content were significantly increased in brain tissues compared to the control group, while TM content was decreased, indicating increase in TM/TDP conversion rate.

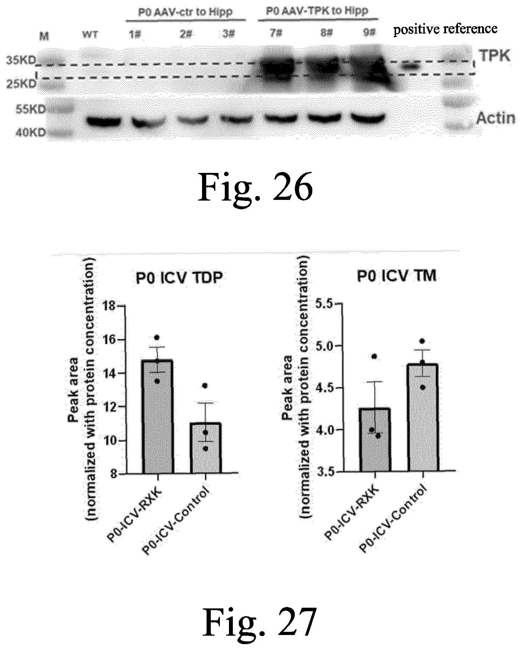

As shown in Fig. 26, 3 weeks after AAV-TPK injection in hippocampus in P0 mice, expression level of TPK in brain tissues was significantly increased compared to the control group.

As shown in Fig. 27, the TM and TDP in liver were not significantly changed, indicating that the virus had good brain specificity, specifically targeting brain neurons without affecting peripheral tissues. The side effects of the virus were completely controllable.

### Example 7: Screening for TPK variants with a high activity

Mutants with higher activity than wild-type TPK polypeptide were screened in this Example by introducing amino acid substitutions into TPK polypeptide.

The present inventors selected amino acids which may have significant impact on TPK activity by comparing the structures of human TPK and mouse TPK proteins, and then the TPK and the mutants thereof as shown in Table 2 were designed and prepared by single mutation and combined mutations on these amino acids.

**Table 2**

| TPK | Sequence (SEQ ID NO:) | Mutation |
|---|---|---|
| human TPK (hTPK) | 8 | N/A |
| hTPK 8M | 9 | S13P-N30A-Y31R-N37K-T85K-A129G-E158K-D181S |
| mouse TPK (mTPK) | 1 | N/A |
| mTPK 11W | 10 | L11W |
| mTPK 35 W | 11 | L35W |
| mTPK 241W | 12 | I241W |
| mTPK 11W+241W | 13 | L11W-I241W |

Specifically, the nucleotide sequences (SEQ ID NO: 1 and 14-19) encoding the TPKs above were cloned into pMCSG7-(+) backbone and transformed into *E. coli.* BL21 (DE3) competent cells. The cells were then spread on LB agar medium (containing 50mg/L of kanamycin) and cultured overnight at 37°C. The single colonies were then transferred to LB liquid medium (containing 50mg/L of kanamycin) for sequencing validation.

The validated clones were activated on LB agar medium. Then, the single colonies were inoculated into LB broth (containing 50mg/L of kanamycin) and incubated at 37°C for 12 h. 1mL of the culture was transferred to 50mL of fresh LB broth (containing 50mg/L of kanamycin) and incubated at 37°C until OD600 reached about 0.6. IPTG (final concentration of 0.5mM) was added and incubated at 25°C for 16 h to induce protein expression.

After incubation, the culture was centrifuged at 5000g, 4°C for 5 min, the supernatants were discarded, and the *E*. *coli* cells were collected. The collected *E*. *coli* cells were resuspended at ratio of 1:8 in pre-cooled protein equilibrium solution (50mM HEPES, 2mM MgCl2, 250mM NaCl, 10% glycerol) and the *E. coli* cells were sonicated at 4°C. Cell fragmentation solution was centrifuged at 12000 rpm, 4° C for 60 min. The supernatant was collected and passed through nickel column (GE, 17-3712-02). The nickel column was cleaned with 30mM imidazole solution prepared with protein equilibrium solution to remove non-specific binding impurity proteins. The nickel column was eluted with 500mM imidazole solution prepared with protein equilibrium solution, and the eluents containing target protein were collected and dialyzed with 10KD aperture concentration tube from Merck Millipore to remove imidazole until its concentration was lower than 5 mM. The dialysis products were further purified through FPLC molecular sieve (SEC column: superdex 200 increase; PBS buffer, pH 7.4), the target protein solution was collected, and the protein was concentrated using 10KD aperture concentration tube from Merck Millipore.

The experimental materials for detecting the enzyme activity were shown in Table 3.

**Table 3**

| Reagent (equipment) name | Vendor | Catalog No. |
|---|---|---|
| Tris-HCl (Trizma hydrochloride) | VETEC | V900312-500G |
| EDTA disodium salt | Sigma | 27285-500G-R |
| 2-mercaptoethanol | Sigma | M6250-100ML |
| MgSO4 | Sigma | 63136-250G-F |
| ATP | Sigma | A26209-5G |
| TM (thiamine hydrochloride) | Sigma | T4625-100G |
| NaOH | Sigma | S8045-500G |
| HCl | Sinopharm/Shanghai Reagent | 10011018 |
| 72% perchloric acid | Sinopharm | 20181207 |
| Distilled water | Watsons | 400 mL/bottle |
| High speed/low temperature centrifuge | Eppendorf | 5430R |
| Thermostatic water bath pot | Shanghai Bilang Instrument Co., Ltd. | DK-8D |
| High performance liquid chromatograph | Shimadzu | Agilent 1260 |

50 mM TPK enzyme solution was mixed with 150 mM Tris HCl buffer and 10 mM ATP solution at equal proportions, and 210 µ L of the mixture was transferred into a centrifuge tube. 30 µ L of DMSO was added to each centrifuge tube, and finally 30 µ L of 20 µ M thiamine solution was added, which was incubated at 37°C for 0.5 h. Then, 270 µ L of 5% PCA quenching solution was added and mixed well to stop the reaction. 150 µL of the above mixture was transferred to a 1.5 mL centrifuge tube, 30 µ L of 10 mM potassium ferricyanide derivatization reagent was added for sample derivatization, and 15 µL of 1M phosphoric acid quenching solution was finally added to stop the reaction. As described in Example 2, TDP/TM contents were detected by high-performance liquid chromatography. TPK enzyme activity = TDP (nM)/mg TPK/min.

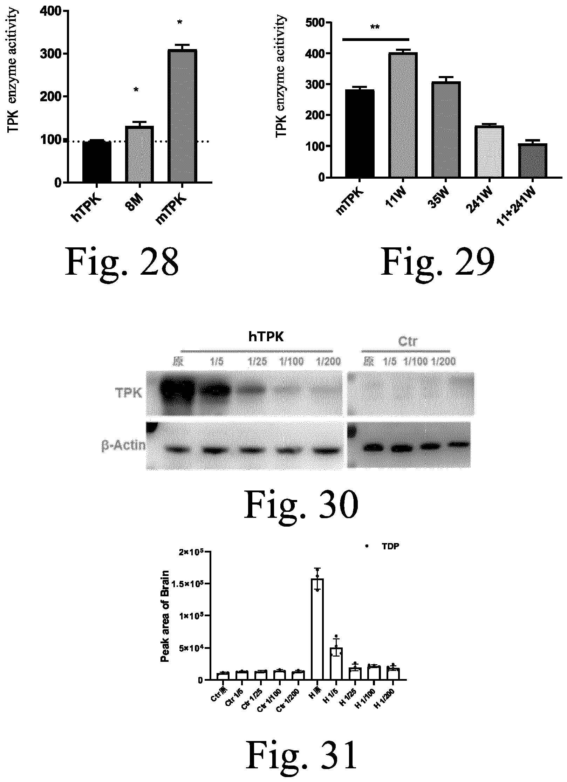

As shown in Fig. 28, compared with human TPK (hTPK), variant hTPK 8M showed significantly increased catalytic activity for TM to TDP conversion (p<0.05), but still lower than that of wild-type mouse TPK (mTPK) (p<0.05). As shown in Fig. 29, compared with wild-type mTPK, the variants mTPK 11W and mTPK 35W showed higher catalytic activity for converting TM to TDP, among which the variant mTPK 11W showed the highest activity (p<0.01 vs. mTPK) while the introduction of 241W substitution lowered the activity.

### Example 8: Effects of rAAV encoding TPK variants on activity of TPK in vivo

The therapeutic effect of rAAV encoding TPK variants with increased activity in neonatal mice and APP/PS1 mice was studied in this Example.

The rAAV encoding hTPK was prepared as described in Example 1, designated as AAV hTPK, of which the genome comprises: i) a neuron-specific promoter (SEQ ID NO: 3), ii) a nucleotide sequence (SEQ ID NO: 14) encoding hTPK and a WPRE element (SEQ ID NO: 4).

### 8.1. TPK overexpression and elevated TM/TDP conversion rate in brain of neonatal mice injected with AAV-hTPK in lateral ventricle

The purpose of this Example is to verify whether direct delivery of AAV-hTPK to lateral ventricle (Intracerebral ventricular, ICV) of neonatal mouse (Postnatal 0 day, P0 mouse) can significantly increase TPK expression in C57BL6/J mouse brain neurons, improve TM/TDP conversion rate in neurons, thereby enhancing glycometabolism.

C57BL6/J mice (embryo 18.5 days, E18.5) were purchased from Shanghai JieSiJie Laboratory Animal Co., Ltd., in total of 11. Total amount of virus stock solution was 2E10vg (800 nL), which was diluted as 1/5, 1/25, 1/100, and 1/200 of the stock solution concentration, respectively. rAAV without TPK encoding sequence was used as control. The experiments were conducted after the birth of the neonatal mice.

Lateral ventricular injection site: 1 mm forward, 1 mm left and right, and 1 mm deep from Lambda. 800 nL of virus suspension was injected in lateral ventricles on both sides.

After the glass electrode was filled with paraffin oil, it was fixed on microinjector with a hot melt gun. Needle of the microinjector was inserted into about one-third in the glass electrode, and the microinjector was fixed to automatic injection pump.

Body of the neonatal mouse was buried in ice without covering its nose to allow breath for about 3 min. After the body changed from pink purple to slightly white without struggle, the P0 mouse was removed from the ice and placed on ice surface for subsequent experiments.

Virus suspension was sucked into glass electrode through injection pump. The neonatal mice deeply anesthetized by ice treatment was fixed on horizontal vessel with medical tapes, keeping the head horizontal.

Under stereomicroscope, the posterior fontanelle (Lambda) of mouse head was designated as the origin, the syringe was moved to the designated position through operating arm, and the up and down positions of the syringe were adjusted through vertical operating arm (Z-axis). The position where the needle slightly sank after touching the head was set as point 0, and the needle was sent downwards to the designated position. Finally, the virus was automatically delivered to brain of neonatal mouse through an injection pump controller. After the injection was completed, the needle was allowed to stay in brain of the neonatal mouse for about 3 min, and then slowly removed.

After injection, the neonatal mouse was quickly transferred to heating pad. After body temperature of the neonatal mouse recovered and it began to move, the animal was placed back into mother mouse cage and covered with cage padding for a period of time to be stained with smell. The stereotactic experiment ended when the mother mouse retrieved the neonatal mouse into the nest initiatively.

After 21 days of feeding, 3 mice of each treatment were treated as described in Example 3 to collect samples and detect expression of TPK expression level and TDP content in brain tissues.

As shown in Fig. 30, the expression of TPK protein in brain tissues showed a dose-dependent change with the titer gradient of the injected virus. HPLC results showed that injection of AAV-hTPK (within the range of stock solution to 100 times dilution) showed a dose-dependent decrease in TDP content in brain tissue as with virus dilution (Fig. 31).

### 8.2. Effect of lateral ventricular injection of AAV-TPK on APP/PS1 mice

The Example verifies whether direct delivery of AAV (AAV-TPK prepared in Example 1) to lateral ventricle (Intracerebral ventricular, ICV) of adult APP/PS1mouse can significantly increase expression of TPK in APP/PS1 mouse brain neurons, improve conversion rate of TM/TDP in neurons, thereby enhancing glycometabolism.

After 3 days of adaptive feeding, adult APP/PS1 male mice (13 months) (purchased from Cavens Biogle(Suzhou) Model Animal Research Co. Ltd.) were randomly divided into two groups (TPK group and control group, n=12) for stereotactic injection of AAV in lateral ventricle. The concentration of virus stock solution used was 1.0E+13vg/lmL, and injection volume in the lateral ventricle was 4.0E+10vg/4µL (2 µL on each side) per mouse. After anesthetizing adult mice with isoflurane, the hair between the ears and eyes was shaved off and the mice were fixed on a brain stereotaxic device. The brain skin of the mice was wiped with an alcohol cotton ball and disinfected. Scissors were used to open the skin and expose the skull. Surface of the skull was wiped to dryness with a dry cotton swab until the anterior fontanelle point was clearly visible. The coordinates of the anterior fontanelle point were designated as 0 (AP: -0.2mm, L/R: ±1.0mm, H: 2.5mm), and the drilling point was marked. A 0.5mm drill bit was used to drill a hole in the skull, and 2 µL of AAV virus was inhaled with a 10 µL micro injection needle. The depth of the needle was 2.5mm, with the skull surface as the Z-axis 0 point. Injection speed: 0.2 µL/min, and the injection was ceased for 5 min, and the injection needle was slowly removed. After the skin was sutured, the mice were placed in a cage containing heating pad and fed in the experimental area of feeding cage for at least 8 weeks after awakening, waiting for the expression of AAV The weight, food intake and blood glucose were recorded weekly (at specified time points).

As described in Example 3, blood, brain tissue, and liver tissue samples were collected to detect TDP and TM levels in the blood, TPK expression levels, and TDP and TM contents in the brain and liver tissues (n=4).

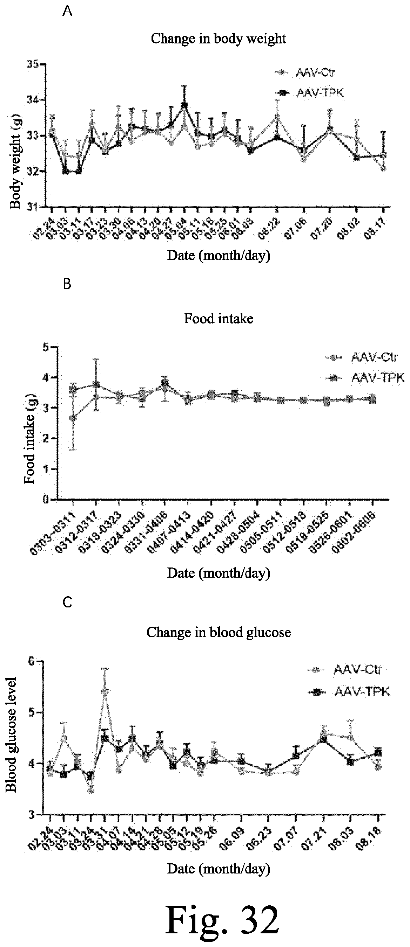

As shown in Fig. 32, there was no significant difference in body weight, food intake, and blood glucose between mice injected with AAV-TPK and those injected with control AAV, indicating the safety of AAV-TPK injection.

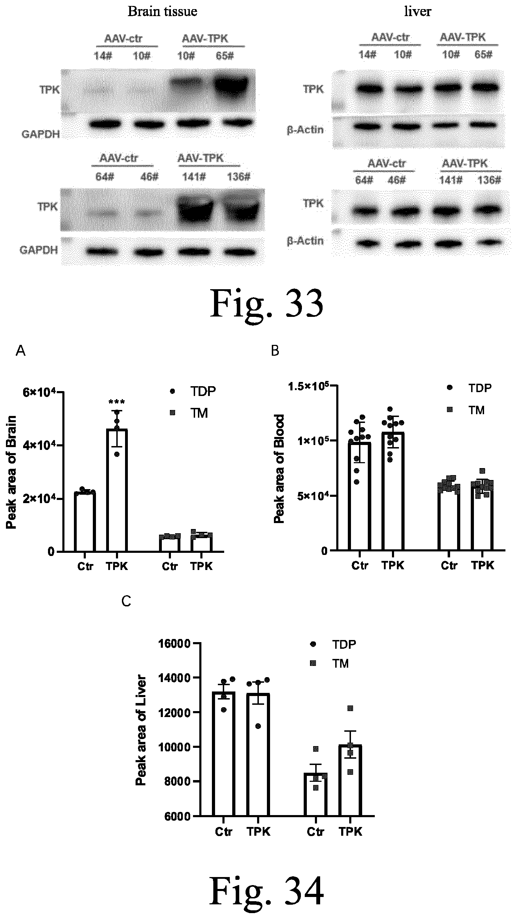

As shown in Fig. 33, the expression level of TPK in brain tissues of mice injected with AAV-TPK was high, while TPK was almost not expressed in brain tissues of mice injected with control AAV; the expression level of TPK in liver of mice injected with AAV-TPK were comparable as those injected with control AAV, indicating that the AAV according to the present disclosure did not show off-target effect.

As shown in Fig. 34, TDP content in brain tissues of APP/PS1 mice injected with AAV-TPK was significantly higher than that of APP/PS1 mice injected with control AAV. TDP contents in blood and liver tissues of APP/PS1 mice injected with AAV-TPK were comparable to those of APP/PS1 mice injected with control AAV, indicating that the AAV according to the present disclosure did not show off-target effect.

After 6 months of feeding, as described in Example 3, the samples were collected and detected (the only difference was the primary antibody) for GSK3β protein level in brain tissues (the primary antibody was GSK-3β (27C10) (purchased from CST, Catalog No. 9315) and phosphorylated GSK-3β protein (p-GSK-3β) (Ser9) (the primary antibody purchased from CST, Catalog No. 14630).

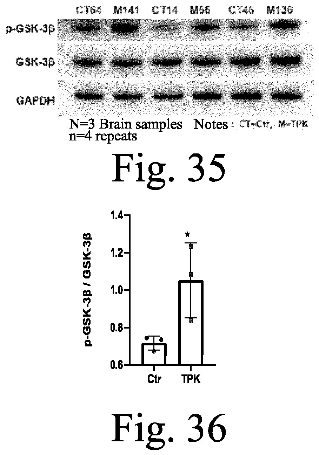

As shown in Fig. 35 and Fig.36, p-GSK3β level and p-GSK3β/GSK3β ratio in brain tissues of APP/PS1 mice inj ected with AAV-TPK in lateral ventricle were higher than those of APP/PS1 mice injected with control AAV, indicating the interaction between "TPK-TDP-glycometabolism pathway" and p-GSK3β pathway.

### Sequence Listing

SEQ ID NO: 1 mouse TPK amino acid sequence 
MEHAFTPLEPLLPTGNLKYCLVVLNQPLDARFRHLWKKALLRACADGGANHLYDLT
EGERESFLPEFVSGDFDSIRPEVKEYYTKKGCDLISTPDQHTDFTKCLQVLQRKIEEK
ELQVDVIVTLGGLGGRFDQIMASVNTLFQATHITPVPIIIIQKDSLIYLLQPGKHRLHVD
TGMEGSWCGLIPVGQPCNQVTTTGLKWNLTNDVLGFGTLVSTSNTYDGSGLVTVET
DHPLLWTMAIKS
SEQ ID NO: 2 mouse TPK nucleotide sequence 
ATGGAGCATGCCTTTACCCCGTTGGAACCCCTGCTACCTACGGGGAACTTGAAATA
CTGCCTTGTGGTTCTTAATCAGCCTTTGGATGCACGATTTCGCCATCTTTGGAAAAA
AGCTCTCTTAAGAGCCTGTGCTGATGGGGGTGCCAACCACTTATATGATCTCACTG
AAGGAGAGAGAGAAAGCTTCTTGCCTGAATTCGTCAGTGGGGACTTTGATTCTATT
AGGCCTGAAGTCAAAGAGTACTACACCAAGAAGGGCTGTGATCTTATTTCAACTC
CTGACCAAGACCACACTGACTTTACCAAGTGTCTTCAAGTGCTCCAAAGGAAGAT
AGAGGAAAAGGAACTGCAGGTTGACGTGATTGTGACACTGGGAGGTCTCGGTGG
GCGTTTTGACCAAATCATGGCCTCTGTGAATACCCTTTTCCAAGCCACTCACATCA
CTCCTGTGCCGATTATAATAATCCAAAAGGACTCTCTCATCTACCTCCTCCAACCCG
GGAAGCACAGGCTCCATGTAGACACTGGAATGGAAGGGAGCTGGTGTGGCCTGAT
TCCTGTTGGACAGCCTTGCAACCAGGTGACGACAACAGGCCTGAAATGGAACCTC
ACAAATGATGTTCTTGGCTTTGGAACACTGGTCAGTACTTCTAACACCTACGATGG
GTCCGGCCTTGTCACTGTGGAAACTGACCACCCACTCCTCTGGACCATGGCCATCA
AGAGCTAA
SEQ ID NO: 3 promoter nucleotide sequence 
AGTGCAAGTGGGTTTTAGGACCAGGATGAGGCGGGGTGGGGGTGCCTACCTGACG
ACCGACCCCGACCCACTGGACAAGCACCCAACCCCCATTCCCCAAATTGCGCATC
CCCTATCAGAGAGGGGGAGGGGAAACAGGATGCGGCGAGGCGCGTGCGCACTGC
CAGCTTCAGCACCGCGGACAGTGCCTTCGCCCCCGCCTGGCGGCGCGCGCCACCG
CCGCCTCAGCACTGAAGGCGCGCTGACGTCACTCGCCGGTCCCCCGCAAACTCCC
CTTCCCGGCCACCTTGGTCGCGTCCGCGCCGCCGCCGGCCCAGCCGGACCGCACC
ACGCGAGGCGCGAGATAGGGGGGCACGGGCGCGACCATCTGCGCTGCGGCGCCG
GCGACTCAGCGCTGCCTCAGTCTGCGGTGGGCAGCGGAGGAGTCGTGTCGTGCCT
GAGAGCGCAG
SEQ ID NO: 4 WPRE nucleotide sequence 
AATCAACCTCTGGATTACAAAATTTGTGAAAGATTGACTGGTATTCTTAACTATGTT
GCTCCTTTTACGCTATGTGGATACGCTGCTTTAATGCCTTTGTATCATGCTATTGCTT
CCCGTATGGCTTTCATTTTCTCCTCCTTGTATAAATCCTGGTTGCTGTCTCTTTATGA
GGAGTTGTGGCCCGTTGTCAGGCAACGTGGCGTGGTGTGCACTGTGTTTGCTGAC
GCAACCCCCACTGGTTGGGGCATTGCCACCACCTGTCAGCTCCTTTCCGGGACTTT
CGCTTTCCCCCTCCCTATTGCCACGGCGGAACTCATCGCCGCCTGCCTTGCCCGCT
GCTGGACAGGGGCTCGGCTGTTGGGCACTGACAATTCCGTGGTGTTGTCGGGGAA
ATCATCGTCCTTTCCTTGGCTGCTCGCCTATGTTGCCACCTGGATTCTGCGCGGGAC
GTCCTTCTGCTACGTCCCTTCGGCCCTCAATCCAGCGGACCTTCCTTCCCGCGGCC
 
TGCTGCCGGCTCTGCGGCCTCTTCCGCGTCTTCGCCTTCGCCCTCAGACGAGTCGG
ATCTCCCTTTGGGCCGCCTCCCCGC
SEQ ID NO: 5 polyadenylation signal sequence of human growth factor gene 
CTGTGCCTTCTAGTTGCCAGCCATCTGTTGTTTGCCCCTCCCCCGTGCCTTCCTTGA
CCCTGGAAGGTGCCACTCCCACTGTCCTTTCCTAATAAAATGAGGAAATTGCATCG
CATTGTCTGAGTAGGTGTCATTCTATTCTGGGGGGTGGGGTGGGGCAGGACAGCA
AGGGGGAGGATTGGGAAGACAATAGCAGGCATGCTGGGGATGCGGTGGGCTCTAT
GG
SEQ ID NO: 6 ITR 
AGGAACCCCTAGTGATGGAGTTGGCCACTCCCTCTCTGCGCGCTCGCTCGCTCACT
GAGGCCGGGCGACCAAAGGTCGCCCGACGCCCGGGCTTTGCCCGGGCGGCCTCA
GTGAGCGAGCGAGCGCGCAG
SEQ ID NO: 7 eukaryotic promoter 
GTGATGCGGTTTTGGCAGTACATCAATGGGCGTGGATAGCGGTTTGACTCACGGGG
ATTTCCAAGTCTCCACCCCATTGACGTCAATGGGAGTTTGTTTTGGCACCAAAATC
AACGGGACTTTCCAAAATGTCGTAACAACTCCGCCCCATTGACGCAAATGGGCGG
TAGGCGTGTACGGTGGGAGGTCTATATAAGCAGAGCT
SEQ ID NO: 8 human TPK amino acid sequence
SEQ ID NO: 9 human TPK 8M amino acid sequence 
MEHAFTPLEPLLPTGNLKYCLVILNQPLDARFRHLWKKALLRACADGGANRLYDITE
GERESFLPEFINGDFDSIRPEVREYYAKKGCELISTPDQDHTDFTKCLKMLQKKIEEKD
LKVDVIVTLGGLGGRFDQIMASVNTLFQATHITPFPIIIIQKESLIYLLQPGKHRLHVDT
GMEGSWCGLIPVGQPCMQVTTTGLKWNLTNDVLAFGTLVSTSNTYDGSGVVTVETD
HPLLWTMAIKS
SEQ ID NO: 10 mouse TPK 11W amino acid sequence 
MEHAFTPLEPWLPTGNLKYCLVVLNQPLDARFRHLWKKALLRACADGGANHLYDLT
EGERESFLPEFVSGDFDSIRPEVKEYYTKKGCDLISTPDQDHTDFTKCLQVLQRKIEEK
ELQVDVIVTLGGLGGRFDQIMASVNTLFQATHITPVPIIIIQKDSLIYLLQPGKHRLHVD
TGMEGSWCGLIPVGQPCNQVTTTGLKWNLTNDVLGFGTLVSTSNTYDGSGLVTVET
DHPLLWTMAIKS
SEQ ID NO: 11 mouse TPK 35W amino acid sequence 
MEHAFTPLEPLLPTGNLKYCLVVLNQPLDARFRHWWKKALLRACADGGANHLYDLT
EGERESFLPEFVSGDFDSIRPEVKEYYTKKGCDLISTPDQDHTDFTKCLQVLQRKIEEK
 
ELQVDVIVTLGGLGGRFDQIMASVNTLFQATHITPVPIIIIQKDSLIYLLQPGKHRLHVD
TGMEGSWCGLIPVGQPCNQVTTTGLKWNLTNDVLGFGTLVSTSNTYDGSGLVTVET
DHPLLWTMAIKS
SEQ ID NO: 12 mouse TPK 241W amino acid sequence 
MEHAFTPLEPLLPTGNLKYCLVVLNQPLDARFRHLWKKALLRACADGGANHLYDLT
EGERESFLPEFVSGDFDSIRPEVKEYYTKKGCDLISTPDQDHTDFTKCLQVLQRKIEEK
ELQVDVIVTLGGLGGRFDQIMASVNTLFQATHITPVPIIIIQKDSLIYLLQPGKHRLHVD
TGMEGSWCGLIPVGQPCNQVTTTGLKWNLTNDVLGFGTLVSTSNTYDGSGLVTVET
DHPLLWTMAWKS
SEQ ID NO: 13 mouse TPK 11W+241W amino acid sequence 
MEHAFTPLEPWLPTGNLKYCLVVLNQPLDARFRHLWKKALLRACADGGANHLYDLT
EGERESFLPEFVSGDFDSIRPEVKEYYTKKGCDLISTPDQDHTDFTKCLQVLQRKIEEK
ELQVDVIVTLGGLGGRFDQIMASVNTLFQATHITPVPIIIIQKDSLIYLLQPGKHRLHVD
TGMEGSWCGLIPVGQPCNQVTTTGLKWNLTNDVLGFGTLVSTSNTYDGSGLVTVET
DHPLLWTMAWKS
SEQ ID NO: 14 human TPK nucleotide sequence 
ATGGAGCATGCCTTTACCCCGTTGGAGCCCCTGCTTTCCACTGGGAATTTGAAGTA
CTGCCTTGTAATTCTTAATCAGCCTTTGGACAACTATTTCGTCATCTTTGGAACAA
AGCTCTTTTAAGAGCCTGTGCCGATGGAGGTGCCAACCGCTTATATGATATCACCG
AAGGAGAGAGAGAAAGCTTTTTGCCTGAATTCATCAATGGAGACTTTGATTCTATT
AGGCCTGAAGTCAGAGAATACTATGCTACTAAGGGATGTGAGCTCATTTCAACTCC
TGATCAAGACCACACTGACTTTACTAAGTGCCTTAAAATGCTCCAAAAGAAGATAG
AAGAAAAAGACTTAAAGGTTGATGTGATCGTGACACTGGGAGGCCTTGCTGGGCG
TTTTGACCAGATTATGGCATCTGTGAATACCTTGTTCCAAGCGACTCACATCACTCC
TTTTCCAATTATAATAATCCAAGAGGAATCGCTGATCTACCTGCTCCAACCAGGAAA
GCACAGGTTGCATGTAGACACTGGAATGGAGGGTGATTGGTGTGGCCTTATTCCTG
TTGGACAGCCTTGTATGCAGGTTACAACCACAGGCCTCAAGTGGAACCTCACAAA
TGATGTGCTTGCTTTTGGAACATTGGTCAGTACTTCCAATACCTACGACGGGTCTG
GTGTTGTGACTGTGGAAACTGACCACCCACTCCTCTGGACCATGGCCATCAAAAG
CTAA
SEQ ID NO: 15 human TPK 8M nucleotide sequence 
ATGGAGCATGCCTTTACCCCGTTGGAGCCCCTGCTTCCTACTGGGAATTTGAAGTA
CTGCCTTGTAATTCTTAATCAGCCTTTGGACGCGCTTTCGTCATCTTTGGAAGAA
AGCTCTTTTAAGAGCCTGTGCCGATGGAGGTGCCAACCGCTTATATGATATCACCG
AAGGAGAGAGAGAAAGCTTTTTGCCTGAATTCATCAATGGAGACTTTGATTCTATT
AGGCCTGAAGTCAGAGAATACTATGCTAAGAAGGGATGTGAGCTCATTTCAACTCC
TGATCAAGACCACACTGACTTTACTAAGTGCCTTAAAATGCTCCAAAAGAAGATAG
AAGAAAAAGACTTAAAGGTTGATGTGATCGTGACACTGGGAGGCCTTGGAGGGC
GTTTTGACCAGATTATGGCATCTGTGAATACCTTGTTCCAAGCGACTCACATCACTC
CTTTTCCAATTATAATAATCCAAAAAGAATCGCTGATCTACCTGCTCCAACCAGGAA
AGCACAGGTTGCATGTAGACACTGGAATGGAGGGTTCTTGGTGTGGCCTTATTCCT
GTTGGACAGCCTTGTATGCAGGTTACAACCACAGGCCTCAAGTGGAACCTCACAA
 
ATGATGTGCTTGCTTTTGGAACATTGGTCAGTACTTCCAATACCTACGACGGGTCTG
GTGTTGTGACTGTGGAAACTGACCACCCACTCCTCTGGACCATGGCCATCAAAAG
CTAA
SEQ ID NO: 16 mouse TPK 11W nucleotide sequence 
ATGGAGCATGCCTTTACCCCGTTGGAACCCTGGCTACCTACGGGGAACTTGAAATA
CTGCCTTGTGGTTCTTAATCAGCCTTTGGATGCACGATTTCGCCATCTTTGGAAAAA
AGCTCTCTTAAGAGCCTGTGCTGATGGGGGTGCCAACCACTTATATGATCTCACTG
AAGGAGAGAGAGAAAGCTTCTTGCCTGAATTCGTCAGTGGGGACTTTGATTCTATT
AGGCCTGAAGTCAAAGAGTACTACACCAAGAAGGGCTGTGATCTTATTTCAACTC
CTGACCAAGACCACACTGACTTTACCAAGTGTCTTCAAGTGCTCCAAAGGAAGAT
AGAGGAAAAGGAACTGCAGGTTGACGTGATTGTGACACTGGGAGGTCTCGGTGG
GCGTTTTGACCAAATCATGGCCTCTGTGAATACCCTTTTCCAAGCCACTCACATCA
CTCCTGTGCCGATTATAATAATCCAAAAGGACTCTCTCATCTACCTCCTCCAACCCG
GGAAGCACAGGCTCCATGTAGACACTGGAATGGAAGGGAGCTGGTGTGGCCTGAT
TCCTGTTGGACAGCCTTGCAACCAGGTGACGACAACAGGCCTGAAATGGAACCTC
ACAAATGATGTTCTTGGCTTTGGAACACTGGTCAGTACTTCTAACACCTACGATGG
GTCCGGCCTTGTCACTGTGGAAACTGACCACCCACTCCTCTGGACCATGGCCATCA
AGAGCTAA
SEQ ID NO: 17 mouse TPK 35W nucleotide sequence 
ATGGAGCATGCCTTTACCCCGTTGGAACCCCTGCTACCTACGGGGAACTTGAAATA
CTGCCTTGTGGTTCTTAATCAGCCTTTGGATGCACGATTTCGCCATTGGTGGAAAA
AAGCTCTCTTAAGAGCCTGTGCTGATGGGGGTGCCAACCACTTATATGATCTCACT
GAAGGAGAGAGAGAAAGCTTCTTGCCTGAATTCGTCAGTGGGGACTTTGATTCTA
TTAGGCCTGAAGTCAAAGAGTACTACACCAAGAAGGGCTGTGATCTTATTTCAACT
CCTGACCAAGACCACACTGACTTTACCAAGTGTCTTCAAGTGCTCCAAAGGAAGA
TAGAGGAAAAGGAACTGCAGGTTGACGTGATTGTGACACTGGGAGGTCTCGGTG
GGCGTTTTGACCAAATCATGGCCTCTGTGAATACCCTTTTCCAAGCCACTCACATC
ACTCCTGTGCCGATTATAATAATCCAAAAGGACTCTCTCATCTACCTCCTCCAACCC
GGGAAGCACAGGCTCCATGTAGACACTGGAATGGAAGGGAGCTGGTGTGGCCTG
ATTCCTGTTGGACAGCCTTGCAACCAGGTGACGACAACAGGCCTGAAATGGAACC
TCACAAATGATGTTCTTGGCTTTGGAACACTGGTCAGTACTTCTAACACCTACGAT
GGGTCCGGCCTTGTCACTGTGGAAACTGACCACCCACTCCTCTGGACCATGGCCAT
CAAGAGCTAA
SEQ ID NO: 18 mouse TPK 241W nucleotide sequence 
ATGGAGCATGCCTTTACCCCGTTGGAACCCCTGCTACCTACGGGGAACTTGAAATA
CTGCCTTGTGGTTCTTAATCAGCCTTTGGATGCACGATTTCGCCATCTTTGGAAAAA
AGCTCTCTTAAGAGCCTGTGCTGATGGGGGTGCCAACCACTTATATGATCTCACTG
AAGGAGAGAGAGAAAGCTTCTTGCCTGAATTCGTCAGTGGGGACTTTGATTCTATT
AGGCCTGAAGTCAAAGAGTACTACACCAAGAAGGGCTGTGATCTTATTTCAACTC
CTGACCAAGACCACACTGACTTTACCAAGTGTCTTCAAGTGCTCCAAAGGAAGAT
AGAGGAAAAGGAACTGCAGGTTGACGTGATTGTGACACTGGGAGGTCTCGGTGG
GCGTTTTGACCAAATCATGGCCTCTGTGAATACCCTTTTCCAAGCCACTCACATCA
 
CTCCTGTGCCGATTATAATAATCCAAAAGGACTCTCTCATCTACCTCCTCCAACCCG
GGAAGCACAGGCTCCATGTAGACACTGGAATGGAAGGAGCTGGTGTGGCCTGAT
TCCTGTTGGACAGCCTTGCAACCAGGTGACGACAACAGGCCTGAAATGGAACCTC
ACAAATGATGTTCTTGGCTTTGGAACACTGGTCAGTACTTCTAACACCTACGATGG
GTCCGGCCTTGTCACTGTGGAAACTGACCACCCACTCCTCTGGACCATGGCCTGG
AAGAGCTAA
SEQ ID NO: 19 mouse TPK 11W+241W nucleotide sequence

ATGGAGCATGCCTTTACCCCGTTGGAACCCTGGCTACCTACGGGGAACTTGAAATA
CTGCCTTGTGGTTCTTAATCAGCCTTTGGATGCACGATTTCGCCATCTTTGGAAAAA
AGCTCTCTTAAGAGCCTGTGCTGATGGGGGTGCCAACCACTTATATGATCTCACTG
AAGGAGAGAGAGAAAGCTTCTTGCCTGAATTCGTCAGTGGGGACTTTGATTCTATT
AGGCCTGAAGTCAAAGAGTACTACACCAAGAAGGGCTGTGATCTTATTTCAACTC
CTGACCAAGACCACACTGACTTTACCAAGTGTCTTCAAGTGCTCCAAAGGAAGAT
AGAGGAAAAGGAACTGCAGGTTGACGTGATTGTGACACTGGGAGGTCTCGGTGG
GCGTTTTGACCAAATCATGGCCTCTGTGAATACCCTTTTCCAAGCCACTCACATCA
CTCCTGTGCCGATTATAATAATCCAAAAGGACTCTCTCATCTACCTCCTCCAACCCG
GGAAGCACAGGCTCCATGTAGACACTGGAATGGAAGGGAGCTGGTGTGGCCTGAT
TCCTGTTGGACAGCCTTGCAACCAGGTGACGACAACAGGCCTGAAATGGAACCTC
ACAAATGATGTTCTTGGCTTTGGAACACTGGTCAGTACTTCTAACACCTACGATGG
GTCCGGCCTTGTCACTGTGGAAACTGACCACCCACTCCTCTGGACCATGGCCTGG
AAGAGCTAA

## Claims

1. A recombinant adeno-associated virus (rAAV) or recombinant lentivirus, comprising in the genome an expression cassette comprising a polynucleotide encoding thiamine pyrophosphokinase (TPK) operatively linked to a promoter.

2. The rAAV or recombinant lentivirus according to claim 1, wherein the TPK comprises an amino acid sequence of SEQ ID NO: 1.

3. The rAAV or recombinant lentivirus according to claim 1 or 2, wherein the polynucleotide comprises a nucleotide sequence of SEQ ID NO: 2.

4. The rAAV or recombinant lentivirus according to any one of claims 1-3, wherein the promoter is a neuron-specific promoter.

5. The rAAV or recombinant lentivirus according to any one of claims 1-4, wherein the promoter comprises a nucleotide sequence of SEQ ID NO: 3.

6. The rAAV or recombinant lentivirus according to any one of claims 1-5, wherein the expression cassette further comprises a WPRE element.

7. The rAAV or recombinant lentivirus according to claim 6, wherein the WPRE element comprises a nucleotide sequence of SEQ ID NO: 4.

8. The rAAV according to any one of claims 1-5, which is an rAAV of serotype AAV_PHP eB.

9. A pharmaceutical composition, comprising the rAAV or recombinant lentivirus according to any one of claims 1-8 and a pharmaceutically acceptable carrier.

10. The pharmaceutical composition according to claim 9, which is formulated for intravenous administration, intracerebral administration, or intrathecal administration.

11. A method for preventing or treating Alzheimer's disease, comprising administering the rAAV or recombinant lentivirus according to any one of claims 1-8, or the pharmaceutical composition according to claim 9 or 10 to a subject in need thereof.

12. The method according to claim 11, wherein the rAAV or recombinant lentivirus or the pharmaceutical composition is administered intravenously, intracerebrally, or intrathecally.

13. Use of the rAAV or recombinant lentivirus according to any one of claims 1-8, or the pharmaceutical composition according to claim 9 or 10 for the manufacture of a medicament for preventing or treating Alzheimer's disease.

14. The use of claim 13, wherein the medicament is administered intravenously, intracerebrally, or intrathecally.

15. A modified thiamine pyrophosphokinase (TPK) polypeptide, comprising, as compared to an initial TPK polypeptide, the substitution of amino acid at one or more positions selected from the group consisting of positions 13, 30, 31, 37, 85, 129, 158, 181, 11 and 35, wherein the amino acid at position 13 is substituted with P, the amino acid at position 30 is substituted with A, the amino acid at position 31 is substituted with R, the amino acid at position 37 is substituted with K, the amino acid at position 85 is substituted with K, the amino acid at position 129 is substituted with G, the amino acid at position 158 is substituted with K, the amino acid at position 181 is substituted with S, the amino acid at position 11 is substituted with W, the amino acid at position 35 is substituted with W,
wherein the modified TPK polypeptide has an improved catalytic activity of converting thiamine (TM) to thiamine diphosphate (TDP) as compared to the initial TPK polypeptide, and
wherein the positions are numbered by reference to SEQ ID NO: 1 or 8.

16. The modified TPK polypeptide according to claim 15, wherein the initial TPK polypeptide is a wildtype TPK polypeptide.

17. The modified TPK polypeptide according to claim 15 or 16, wherein the initial TPK polypeptide is human TPK polypeptide, and wherein the modified TPK polypeptide comprises the substitution of amino acid at one or more positions selected from the group consisting of positions 13, 30, 31, 37, 85, 129, 158 and 181, preferably all of positions 13, 30, 31, 37, 85, 129, 158 and 181.

18. The modified TPK polypeptide according to claim 15 or 16, wherein the initial TPK polypeptide is mouse TPK polypeptide, and the modified TPK polypeptide comprises the substitution of amino acid at positions 11 and/or 35.

19. The modified TPK polypeptide according to any one of claims 15-18, comprising an amino acid sequence of SEQ ID NO: 9, 10 or 11.

20. A polynucleotide, encoding the modified TPK polypeptide according to any one of claims 15-19.

21. An expression cassette, comprising the polynucleotide according to claim 20, operatively linked to a promoter.

22. The expression cassette according to claim 21, wherein the promoter is a neuron-specific promoter.

23. The expression cassette according to claim 21 or 22, wherein the promoter comprises a nucleotide sequence of SEQ ID NO: 3.

24. The expression cassette according to any one of claims 21-23, further comprising a WPRE element.

25. The expression cassette according to claim 24, wherein the WPRE element comprises a nucleotide sequence of SEQ ID NO: 4.

26. An rAAV or recombinant lentivirus, comprising the expression cassette according to any one of claims 21-25 in the genome.

27. The rAAV according to claim 26, which is a rAAV of serotype AAV_PHP eB.

28. A pharmaceutical composition, comprising the rAAV or recombinant lentivirus according to claim 26 or 27, and a pharmaceutically acceptable carrier.

29. The pharmaceutical composition according to claim 28, which is formulated for intravenous administration, intracerebral administration, or intrathecal administration.

30. A method for treating or preventing Alzheimer's disease, comprising administering a subject in need thereof the TPK polypeptide according to any one of claims 15-19, the polynucleotide according to claim 20, the AAV or recombinant lentivirus according to claim 26 or 27, or the pharmaceutical composition according to claim 28 or 29.

31. The method according to claim 30, wherein the polypeptide, polynucleotide, rAAV or recombinant lentivirus, or pharmaceutical composition is administered intravenously, intracerebrally, or intrathecally.

32. Use of the TPK polypeptide according to any one of claims 15-19, the polynucleotide according to claim 20, the AAV or recombinant lentivirus according to claim 26 or 27, or the pharmaceutical composition according to claim 28 or 29 for the manufacture of a medicament for treating or preventing Alzheimer's disease.

33. The use according to claim 32, wherein the wherein the medicament is administered intravenously, intracerebrally, or intrathecally.
